(19) Europäisches Patentamt
European Patent Office
Office européen des brevets

(11) **EP 4 129 259 B1**

(12) **EUROPEAN PATENT SPECIFICATION**

(45) Date of publication and mention
of the grant of the patent:
**16.04.2025   Bulletin 2025/16**

(21) Application number: **21189653.5**

(22) Date of filing: **04.08.2021**

(51) International Patent Classification (IPC):
*A61F 13/513* [(2006.01)]      *A61F 13/537* [(2006.01)]
*A61F 13/84* [(2006.01)]

(52) Cooperative Patent Classification (CPC):
**A61F 13/513; A61F 13/537; A61F 13/8405;**
A61F 2013/530708; A61F 2013/8411;
A61F 2013/8435

(54) **ABSORBENT ARTICLE WITH UREASE INHIBITOR AND USE OF THE ARTICLE**

ABSORBIERENDER ARTIKEL MIT UREASE-INHIBITOR UND VERWENDUNG DES ARTIKELS

ARTICLE ABSORBANT AVEC INHIBITEUR DE L'URÉE ET UTILISATION DE L'ARTICLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.02.2023   Bulletin 2023/06**

(73) Proprietor: **The Procter & Gamble Company
Cincinnati, OH 45202 (US)**

(72) Inventors:
 • **EHRNSPERGER, Bruno Johannes
65824 Schwalbach am Taunus (DE)**
 • **MATHUR, Deepika
65824 Schwalbach am Taunus (DE)**
 • **CHATTERJEE, Aniruddha
65824 Schwalbach am Taunus (DE)**
 • **SCHMIDT, Mattias
65824 Schwalbach (DE)**
 • **KAMPHUS, Juliane
65824 Schwalbach am Taunus (DE)**

(74) Representative: **P&G Patent Germany
Procter & Gamble Service GmbH
Sulzbacher Straße 40
65824 Schwalbach am Taunus (DE)**

(56) References cited:
CN-A- 110 946 712      KR-A- 20190 002 144
US-A1- 2003 082 219     US-A1- 2013 096 526
US-A1- 2020 060 897

**Description**

FIELD OF THE INVENTION

The present invention relates to absorbent articles comprising a urease inhibitor, wherein the urease inhibitor is provided underneath the topsheet. The invention also relates to the use of an absorbent article with a urease inhibitor underneath the topsheet, to maintain the pH of the wearer's skin in a range of 4.0 to 6.0.

BACKGROUND OF THE INVENTION

Absorbent articles, such as diapers and pants, are well known. While their use is widely accepted, some wearers or caretakers have concerns regarding skin health when using such articles. Skin irritation associated with absorbent articles is often referred to as "diaper rash". Especially for delicate skin of babies, skin health is important. However, skin health is generally a focus for many consumers, also when the absorbent articles are used on toddlers and adults.

Skin irritation can inter alia be caused by high levels of humidity inside the absorbent article during use, and by urine and feces deposited inside the absorbent article. The likelihood of skin irritation thereby increases with prolonged wearing time of the absorbent article.

A known means to address and improve skin health, is the application of lotion on the body-facing surface of the topsheet of the absorbent article. Such lotions often comprise skin-care components. During use of the article, at least a part of the lotion is intended to transfer to the wearer's skin, to provide a certain protection against skin irritation.

However, due to its typically hydrophobic nature, the application of lotion on the topsheet of an absorbent article may negatively impact the acquisition of liquid (such as urine) through the topsheet. Also, a part of the lotion may migrate through the topsheet into the absorbent article during use - or even during storage of the absorbent article prior to use, especially at higher temperatures. Once inside the absorbent article, the lotion can further adversely impact proper acquisition and storage of urine e.g., within the absorbent material of the absorbent core. Consequently, the use of lotion or other (hydrophobic) substances on the body-facing surface of the topsheet may not only have positive effects on skin care but may also result in some effects which negatively impact skin health, e.g., by prolonging the time which the urine stays on the topsheet before being absorbed through the topsheet, or by slower liquid acquisition and storage inside the absorbent article.

Thus, there remains a need for absorbent articles which can help to reduce the likelihood of diaper rashes.

US2020060897 relates to a fluid-absorbent article includes an upper liquid-pervious layer (A), a lower liquid-impervious layer (B), a fluid-absorbent core (C) between (A) and (B) including at least 60% by weight of non-surface postcrosslinked fluid-absorbent polymer particles and not more than 40% by weight of fibrous material. An acquisition-distribution layer (D) between (A) and (C) includes at least 90% by weight of synthetic fibers and not more than 10% by weight of cellulose based fibers.

US2013096526 discloses fluid-absorbent articles, comprising a fluid-absorbent core comprising at least 60% by weight of fluid-absorbent polymer particles and not more than 40% by weight of cellulose based fibers and an acquisition-distribution layer comprising at least 90% by weight of synthetic non-cellulose based fibers and not more than 10% by weight of cellulose based fibers.

CN110946712 is concerned with an infant paper diaper comprising a surface coating layer, an absorption core layer and a bottom film. The surface coating layer contains skin care components and components for controlling the pH value are added into the absorption core layer.

US2003082219 relates to skin care compositions that are suitable for application on absorbent articles. The skin care compositions comprise low concentrations of skin treatment agents that are effective in preventing and/or reducing skin disorders related to erythema, malodor, and skin bacterial infections.

KR20190002144 is concerned with a pad comprising lavender extract, rosemary extract and chamomile extract and a method manufacturing thereof.

SUMMARY OF THE INVENTION

Skin health and protection from biological insults are important for wearers of absorbent articles. Absorbent articles such as diapers, pants, adult incontinence products, are worn such that they are in direct contact with the skin of the wearer. An unavoidable consequence of the use of absorbent articles is that the skin is exposed more directly to various physical and biological insults. Consequently, the barrier function of the skin covered by the absorbent article is put at risk.

The decay of urea, which has entered the absorbent article, such as a diaper or pant, leads to the formation of ammonia. The decay of urea is catalyzed by the enzyme urease, which is produced by bacteria present on skin and in feces. Therefore, inhibiting urease activity inside the absorbent article, can help to reduce the degree of ammonia formation. It has been found that ammonia elevates the skin pH.

The pH value of skin should be between 4.5 and 6, or optimally between 4.7 and 5.75. Hence, skin's natural pH is mildly acidic. This mildly acidic pH is created by the skin's acid mantle, the water part of the hydrolipid film that protects the external layers of skin.

Skin's pH has been found to play an important role in skin condition. The acid mantle is key to skin's protective barrier. For example, it inhibits the growth of bacteria and restores and maintains the optimal acid environment in which skin's natural flora can thrive. If skin's pH rises into the alkaline range, its natural balance is disturbed. Essential epidermal lipids cannot be synthesized, and skin loses water and dries out. In this condition, the outer layer of skin (or epidermis) is no longer able to work as a protective barrier.

When skin's barrier function is compromised it is less resilient and more sensitive to environmental triggers. It can become dry, sensitive or hypersensitive, and so-called diaper rash can occur if this happens in the area of the skin which is covered by the absorbent article.

It is thus very desirable to maintain the pH of the skin that comes into contact with the absorbent article, within the range of pH 4.5 to 6. Given that it has been found that elevated levels of ammonia contribute to an increase of skin pH, one way of helping the maintenance of the mildly acidic skin pH, is to maintain low ammonia levels inside the diaper. This, in turn, can be achieved by reducing the degree of urea decay inside the absorbent article during use, such as by providing urease inhibitors.

The inventors have found that such urease inhibitors do not have to be applied close to the wearer's skin, such as by incorporating the urease inhibitors in a lotion that is applied on the body-facing surface of the topsheet, or by otherwise providing the urease inhibitors on the body-facing surface of the topsheet. Instead, it has been found that the urease inhibitors can be provided underneath the topsheet, namely between the topsheet and the absorbent core and/or within the absorbent core.

While urine and feces initially get into contact with the body-facing surface of the topsheet, it has been found that the urease inhibitor does not need to be provided on the topsheet's wearer facing surface to be able to be immersed in the urine. Instead, providing the urease inhibitor deeper inside the absorbent article and thus underneath the topsheet, positions the urease inhibitor in the location or close to the location where the urine gets ultimately stored (typically within the absorbent core).

Moreover, providing the urease inhibitors away from the skin-contacting surface of the absorbent article, eliminates or largely reduces the urease inhibitors which get into direct contact with the skin of the wearer. While suitable urease inhibitors are not harmful for the skin, reducing the number of different substances and compounds that are transferred to skin, may be appreciated by users, as exposing the skin to a large variety of different substances may be stressful for the skin.

The present invention relates to the use of a urease inhibitor, in an absorbent article to maintain the pH of the wearer's skin in a range of 4.0 to 6.0, preferably in a range of 4.5 to 6 according to the Urease Activity test method set out herein. The urease inhibitor are thus comprised by the absorbent article in an effective amount.

More than one urease inhibitor may be used in the absorbent article. If more than one urease inhibitor is used, the term "a urease inhibitor" or "the urease inhibitor" refers to all urease inhibitors used, unless specifically noted otherwise. Also, if more than one urease inhibitor is used, any amount of urease inhibitor mentioned herein refers to the total amount of all urease inhibitors used, unless specifically noted otherwise.

The absorbent article comprises a topsheet, a backsheet and an absorbent core provided between the topsheet and the backsheet.

The urease inhibitor is provided between the topsheet and the absorbent core and/or is provided in the absorbent core.

The invention further relates to an absorbent article comprising a topsheet, a backsheet and an absorbent core provided between the topsheet and the backsheet. A urease inhibitor is provided between the topsheet and the absorbent core and/or is provided in the absorbent core.

The absorbent article has a longitudinal centerline and longitudinal dimension extending along the longitudinal centerline, and a transverse centerline perpendicular to the longitudinal centerline.

The absorbent article comprises a front waist region with a front waist edge, a back waist region with a back waist edge, and a crotch region longitudinally extending between the front and back waist region. The absorbent article has a longitudinal dimension extending along the longitudinal centerline from the front waist edge to the rear waist edge, each region forming one third of the longitudinal dimension.

The amount of the urease inhibitor is higher in the crotch region than in the back waist region.

The urease inhibitor comprised by the absorbent article in an effective amount

The absorbent article may comprise more than one urease inhibitor. If the absorbent article comprises more than one urease inhibitor, the term "a urease inhibitor" or "the urease inhibitor" refers to all urease inhibitors in the absorbent article, unless specifically noted otherwise. Also, if the absorbent article comprises more than one urease inhibitor, any amount of urease inhibitor mentioned herein refers to the total amount of all urease inhibitors, unless specifically noted otherwise.

The urease inhibitor of the present invention may comprise a natural urease inhibitor or may, preferably, consist of a natural urease inhibitor.

A natural urease inhibitor is obtained from natural sources, such as green tea or grapeseed.

BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:

Fig. 1 is a plan view of an example absorbent article in the form of a taped diaper, garment-facing surface facing the viewer, in a flat laid-out state;
Fig. 2 is a plan view of the example absorbent article of Fig. 1, body-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a rear perspective view of the absorbent article of Fig. 4;
Fig. 6 is a plan view of the absorbent article of Fig. 4, laid flat, with a garment-facing surface facing the viewer;
Fig. 7 is a cross-sectional view of the absorbent article taken about line 7-7 of Fig. 6;
Fig. 8 is a cross-sectional view of the absorbent article taken about line 8-8 of Fig. 6;
Fig. 9 is a plan view of an example absorbent core or an absorbent article;
Fig. 10 is a cross-sectional view, taken about line 10-10, of the absorbent core of Fig. 9;
Fig. 11 is a cross-sectional view, taken about line 11-11, of the absorbent core of Fig. 10;
Fig. 12 is a plan view of an example absorbent article of the present disclosure that is a sanitary napkin;
Fig. 13 is an example cross-sectional view taken within a front waist region of an absorbent article;
Fig. 14 is an example cross-sectional view taken within a crotch region of an absorbent article;
Fig. 15 is an example cross-sectional view taken within a back waist region of an absorbent article;
Fig. 16 illustrates an apparatus used in the Modified Fluid Acquisition Test;
Fig. 17A is a side view of the curved component used in the Modified Fluid Acquisition Test;
Fig. 17B is an end view of the curved component of FIG. 17A;
Fig. 17C is a bottom view of the curved component of FIG. 17A;
Fig. 17D is a bottom perspective view of the curved component of FIG. 17A;
Fig. 17E is a top perspective view of the curved component of FIG. 17A;
Fig. 18A illustrates a top plate assembly used in the Modified Fluid Acquisition Test;
Fig. 18B illustrates equipment used in the Modified Fluid Acquisition Test;
Fig. 19 shows an equipment assembly used in the Post Acquisition Collagen Rewet Test Method;
Fig. 20 shows an equipment assembly used in the Fixed Height Frit Absorption (FHFA) Test Methods;
Figs. 21 and 22 show partially schematic views of an equipment assembly used in the In-Plane Radial Permeability (IPRP) Test described herein.

DETAILED DESCRIPTION OF THE INVENTION

Definitions

"Absorbent article" refers to devices that absorb and contain body exudates, particularly urine and other water-containing liquids, and, more specifically, refers to devices that are placed against or in proximity to the body of the wearer to absorb and contain the various exudates discharged from the body. Absorbent articles may include diapers (diapers for babies and infants and diapers to address adult incontinence), pants (pants for babies and infants and pants to address adult incontinence), disposable absorbent inserts for diapers and pants having a re-usable outer cover), feminine care absorbent articles such as sanitary napkins or pantiliners. As used herein, the term "exudates" includes, but is not limited to, urine, blood, vaginal discharges, sweat and fecal matter. Preferred absorbent articles of the present invention are diapers, pants and inserts. Also, preferred absorbent articles of the present invention are disposable absorbent articles, more preferably disposable diapers, disposable pants and disposable absorbent inserts.

"Absorbent core" is used herein to refer to a structure intended to be disposed between a topsheet and backsheet of an absorbent article for absorbing and storing liquid received by the absorbent article.

"Airfelt" is used herein to refer to comminuted wood pulp, which is a form of cellulosic fiber.

"Disposable" is used in its ordinary sense to mean an article that is disposed or discarded after a limited number of usage events over varying lengths of time, for example, less than 10 events, less than 5 events, or less than 2 events. If the disposable absorbent article is a diaper, a pant, absorbent insert, sanitary napkin, sanitary pad or wet wipe for personal hygiene use, the disposable absorbent article is most often intended to be disposed after single use. The used and disposed absorbent article may or may not be subsequently recycled.

"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be pre-formed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

"Superabsorbent polymer material" ("SAP material") is used herein to refer to cross-linked polymeric materials that can absorb at least 7 times their weight of an aqueous 0.9 weight- % saline solution as measured using the Centrifuge Retention Capacity test set out below. Superabsorbent polymer material of the present invention contains polymers that comprise as monomer groups acrylic acid and/or acrylate and/or methacrylic acid and/or methacrylate. The SAP material is preferably provided in the form of superabsorbent polymer particles (SAP particles) and/or in the form of superabsorbent fibers (SAF).

"Superabsorbent polymer particles" ("SAP particles") is used herein to refer to superabsorbent polymer material that is in particulate form so as to be flowable in the dry state. Superabsorbent polymer particles are distinguished from the superabsorbent fibers of the present invention in that their ratio of largest to smallest dimension is not more than 10 to 1. Superabsorbent polymer particles may for example be in the form of granules, spheres, flakes or agglomerates.

As used herein, the term "nonwoven web" refers to a material which is a manufactured web/layer of directionally or randomly oriented fibers or filaments. The fibers may be of natural or man-made origin. Natural fibers may be selected from the group consisting of wheat straw fibers, rice straw fibers, flax fibers, bamboo fibers, cotton fibers, jute fibers, hemp fibers, sisal fibers, bagasse fibers, Hesper aloe fibers, miscanthus, marine or fresh water algae/seaweeds, silk fibers, wool fibers, and combinations thereof. Another group of fibers may also be regenerated cellulose fibers, such as viscose, Lyocell (Tencel®), rayon, modal, cellulose acetate fibers, acrylic fibers, cuprammonium rayon, regenerated protein fibers etc. Preferably, the natural fibers or modified natural fibers are selected from the group consisting of cellulose fibers (also referred to as pulp or airfelt) or modified cellulose fibers, such as intra-fiber crosslinked cellulose fibers, cotton fibers, bamboo fibers, viscose fibers or mixtures thereof. More preferably, the natural fibers or modified natural fibers are cellulose fibers or modified cellulose fibers. Synthetic fibers may be selected from the group consisting of polyolefins (such as polyethylene, polypropylene or combinations and mixtures thereof), polyethylene terephthalate (PET), co-PET, polylactic acid (PLA), polybutylene succinate (PBS), polyhydroxy alkanoid (PHA), nylon (or polyamide), or mixtures or combinations thereof.

The fibers in a nonwoven web are consolidated by friction, and/or entanglement, and/or cohesion, and/or adhesion, and/or by heat bonding, pressure bonding, or heat and pressure bonding, and/or ultrasonic bond, excluding paper and products which are woven, knitted, tufted, stitch-bonded. The fibers may be staple fibers (e.g. in carded nonwoven webs) or continuous fibers (e.g. in spunbonded or meltblown nonwoven webs).

The nonwoven webs comprised by the absorbent core of the present invention may comprise or may consist of superabsorbent fibers.

Nonwoven webs can be formed by many processes such as meltblowing, spunlaying, solvent spinning, electrospinning, and carding, and the fibers can be consolidated, e.g. by hydroentanglement (in spunlace nonwoven webs), air-through bonding (using hot air that is blown through the fiber layer in the thickness direction), infrared heat, needle-punching, one or more patterns of bonds and bond impressions created through localized compression and/or application of heat or ultrasonic energy, or a combination thereof. The fibers may, alternatively or in addition, be consolidated by use of a binder. The binder may be provided in the form of binder fibers or particles (which are subsequently molten) or may be provided in liquid, such as a styrene butadiene binder. A liquid binder is provided to the fibers (e.g. by spraying, printing or foam application) and is subsequently cured to solidify.

The basis weight of nonwoven webs is usually expressed in grams per square meter (g/m$^2$).

The nonwoven web, especially nonwoven webs consisting of or comprising superabsorbent fibers, may be carded webs formed by needle-punching. In needle punching, the fibers cohesion and the interlacing of the fibers with one another is obtained by means of needles passing through a moving fibrous layer and causing the fibers to intermingle with one another. One or more, or all of the nonwoven webs of the absorbent core of the present invention may also be formed of two or more precursor webs, which are combined with each other by a needle punching process.

Alternatively, one or more, or all of the nonwoven webs of the absorbent core of the present invention may be formed by spunlacing. In a spunlace nonwoven web the fibers have been carded as precursor web and then subjected to hydroentanglement to intermingle and intertwine the fibers with each other. Cohesion and the interlacing of the fibers with one another may be obtained by means of a plurality of jets of water under pressure passing through a moving fleece or cloth and, like needles, causing the fibers to intermingle with one another (hereinafter also referred to as "hydraulic interlacing"). Thus, consolidation of a spunlace nonwoven web is essentially a result of hydraulic interlacing. "Spunlace nonwoven web", as used herein, also relates to a nonwoven web formed of two or more precursor webs, which are combined with each other by hydraulic interlacing.

The two or more webs, prior to being combined into one nonwoven by needle-punching or hydraulic interlacing, may have undergone bonding processes, such as heat and/or pressure bonding by using e.g. a patterned calendar roll and an anvil roll to impart a bonding pattern. However, the two or more webs are combined with each other solely by needle-punching or hydraulic interlacing.

Alternatively, the carded nonwoven web made by needle-punching or spunlacing is a single nonwoven web, i.e., it is not formed of two or more precursor webs. Still in another alternative, one or more, or all of the nonwoven webs of the absorbent core of the present invention may be formed of one precursor web onto which staple fibers are laid down. The staple fibers may be superabsorbent fibers or may comprise superabsorbent fibers. The staple fibers may not have been consolidated into a self-sustaining precursor web but the fibers are loosely laid onto the precursor web. The relatively loose staple fibers are then integrated and intertwined with each other and with the fibers of the underlying precursor web by (only) needle-punching or (only) hydraulic interlacing. Spunlace and/or needle punched nonwoven layers/webs can be made of staple fibers or continuous fibers (continuous fibers are also often referred to as filaments).

Through-air bonding (interchangeably used with the term "air-through bonding") means a process of bonding staple fibers or continuous fibers by forcing air through the nonwoven web, wherein the air is sufficiently hot to melt (or at least partly melt, or melt to a state where the fiber surface becomes sufficiently tacky) the polymer of a fiber or, if the fibers are multicomponent fibers, wherein the air is sufficiently hot to melt (or at least partly melt, or melt to a state where the fiber surface becomes sufficiently tacky) one of the polymers of which the fibers of the nonwoven web are made. The melting and re-solidification of the polymer provide the bonding between different fibers.

"Comprise," "comprising," and "comprises" are open ended terms, each specifies the presence of the feature that follows, e.g., a component, but does not preclude the presence of other features, e.g., elements, steps, components known in the art or disclosed herein. These terms based on the verb "comprise" encompasses the narrower terms "consisting essentially of" which excludes any element, step or ingredient not mentioned which materially affect the way the feature performs its function, and the term "consisting of" which excludes any element, step, or ingredient not specified.

"Body-facing" (also referred to as "skin-facing" herein) and "garment-facing" refer respectively to the relative location of an element or a surface of an element, layer, component. "Body-facing" implies the element or surface is nearer to the wearer during wear than another element of the same component. An example is the absorbent core having a body-facing surface, which is the surface of the absorbent core that is nearer to the body of the wearer than the opposite surface of the absorbent core, which is garment-facing. "Garment-facing" implies the element or a surface of an element, layer, component, is more remote from the wearer during wear than another element or a surface of an element, layer, component, or of the absorbent article as a whole. The garment-facing surface may face another (i.e. other than the wearable article) garment of the wearer, other items, such as the bedding, or the atmosphere. Typically, the body-facing surface of the topsheet forms a least a portion of the body-facing surface of the absorbent article as a whole, and the garment-facing surface of the outer cover nonwoven forms at least a portion of the garment-facing surface of the absorbent article as a whole.

"Effective amount", as used herein, means the amount of urease inhibitor that is required to be provided in the absorbent article in order to maintain the pH of the wearer's skin in a range of 4.0 to 6, as is measured by the test method set out herein. While the test method is not carried out on a wearer, the test method has been carefully set up to "mimic" as closely as possible the conditions as they typically occur during use of an absorbent article. Indeed, the test method is supposed to provide a more objective measure as it excludes various potential other factors that may impact the pH of a wearer's skin.

The "wearer's skin" as used herein refers to those parts of the skin which are covered by the absorbent article. In the test method below, the collagen is used to represent the wearer's skin.

Use of a urease inhibitor in an absorbent article and absorbent article comprising a urease inhibitor

The invention relates to the use of a urease inhibitor in an absorbent article to maintain the pH of the wearer's skin in a range of 4.0 to 6.0 according to the Urease Activity test method set out herein. As is already said above, while the Urease Activity test method is not carried out on a wearer and thus does not comprise any steps that bring the absorbent article into contact with a wearer's skin, the Urease Activity test method has been carefully set up to "mimic" as closely as possible the conditions as they typically occur during use of an absorbent article. Indeed, the Urease Activity test method is supposed to provide a more objective measure as it excludes various potential other factors that may impact the pH of a wearer's skin.

The absorbent article, wherein the urease inhibitor is used, comprises a liquid permeable topsheet, a liquid impermeable backsheet and an absorbent core provided between the topsheet and the backsheet. The urease inhibitor is provided between the topsheet and the absorbent core and/or is provided in the absorbent core. Hence, the urease inhibitor is not provided on the body-facing surface of the absorbent article (which is the body-facing surface of the topsheet). Though less preferred, being provided between the topsheet and the absorbent core included the provision of the urease inhibitor on the garment-facing surface of the topsheet.

The urease inhibitor may further be used to maintain the pH of the topsheet in a range of 5.0 to 7.5, or in the range of 5.0 to 7.0 according to the test method set out herein.

The urease inhibitor may further be used to maintain the amount of ammonia below 10 ppm, or below 8 ppm, or below 5 ppm, according to the Urease Activity test method set out herein.

While the use of urease inhibitors, such as green tea, on the topsheet of an absorbent article is known, e.g., where a green tea extract is applied in a lotion on a topsheet, the amounts of these compounds in the lotion are typically very low. Such low amounts may not be sufficient to obtain the desired result, namely the maintenance of the skin pH in the desired range.

Amounts of urease inhibitor required to use the urease inhibitor to maintain the pH of the wearer's skin in the desired range may become challenging when incorporated e.g. in the lotion. Moreover, lotion provided on the topsheet of an absorbent article, is typically hydrophobic and contains very little to no water. The urease inhibitor may thus be "trapped" in the lotion which may limit its ability to reduce the decay of urea is by the enzyme urease. Hence, also higher amounts of urease inhibitor may not be sufficient to maintain the pH of the wearer's skin in the desired range.

Applying the urease inhibitor underneath the topsheet makes it much easier to raise the amount of urease inhibitor to a level where it can actually contribute to the maintenance of the skin pH in a range of 4.5 to 6.0.

The urease inhibitor may a natural urease inhibitor. Urease inhibitors and natural urease inhibitors are described in more detail below.

The absorbent core may comprise a surface area facing towards the topsheet (also the body-facing surface of the absorbent core). This body-facing surface of the absorbent core has a certain surface area (which can be easily determined using a ruler).

Generally, the urease inhibitor is applied in an effective amount in order to maintain the pH of the skin within 4.5 and 6, according to the Urease Activity test method set out below. The urease inhibitor may be applied in an amount of at least 3 $g/m^2$, or at least 5 $g/m^2$, or at least 8 $g/m^2$, or at least 10 $g/m^2$, or at least 12 $g/m^2$ based on the total body-facing surface area of the absorbent core. The urease inhibitor may be applied in an amount of not more than 200 $g/m^2$, or not more than 150 $g/m^2$, or not more than 120 $g/m^2$, or not more than 100 $g/m^2$, or not more than 80 $g/m^2$ based on the total body-facing surface area of the absorbent core. For absorbent articles wherein the urease inhibitor is not distributed homogeneously, these amounts of urease inhibitor are the average amounts based on the total body-facing surface area of the absorbent core. The fact that the amounts are put in relation to the body-facing surface area of the absorbent core, does not mean that the urease inhibitor needs to actually be provided on the surface area of the absorbent core. Instead, the urease inhibitor can be provided in several different locations as described and specified herein.

The absorbent article has a longitudinal centerline and longitudinal dimension extending along the longitudinal centerline, and a transverse centerline perpendicular to the longitudinal centerline. The absorbent artic comprises a front waist region with a front waist edge, a back waist region with a back waist edge, and a crotch region longitudinally extending between the front and back waist region. The absorbent article has a longitudinal dimension extending along the longitudinal centerline from the front waist edge to the rear waist edge. The front waist region, the back waist region and the crotch region each form one third of the longitudinal dimension of the absorbent article.

The amount of the urease inhibitor is higher in the crotch region than in the back waist region. The amount of the urease inhibitor may be at least 10 weight-%, or at least 20 weight- %, or at least 50 weight-%, or at least 100 weight-% higher in the crotch region than in the back waist region. The amount of the urease inhibitor may be not more than 300 weight-% higher in the crotch region than in the back waist region.

The amount of the urease inhibitor may be higher in the front waist region than in the back waist region. The amount of the urease inhibitor may be at least 10 weight-%, or at least 20 weight- %, or at least 50 weight-%, or at least 100 weight-% higher in the front waist region than in the back waist region. The amount of the urease inhibitor may be not more than 250 weight-% higher in the front waist region than in the back waist region.

The amount of the urease inhibitor may be higher in the crotch region than in the front waist region. The amount of the urease inhibitor may be at least 3 weight-%, or at least 5 weight- %, or at least 8 weight-% higher in the crotch region than in the front waist region. The amount of the urease inhibitor may be not more than 25 weight-%, or not more than 20 weight-% higher in the crotch region than in the front waist region.

The absorbent core may comprise a front region with a front edge, a back region with a back edge, and a crotch core region longitudinally extending between the front and back region of the absorbent core. The front region, the back region and the crotch core region may each region form one third of the longitudinal dimension of the absorbent core.

The absorbent article may further comprise at least one acquisition and distribution layer (ADL) which is provided between the topsheet and the absorbent core. The ADL may comprise or consist of a first layer and a second layer. The urease inhibitor may be applied a) between the topsheet and the ADL, b) in the ADL, such as between the first and second layer of the ADL, c) between the ADL and the absorbent core, d) in the absorbent core; or e) any combination of a) to d).

Nowhere may the ADL and any layer thereof be longer than the absorbent core along the longitudinal dimension of the absorbent article. Also, nowhere may the ADL and any layer thereof be wider than the absorbent core along the transverse dimension of the absorbent article.

If the urease inhibitor is comprised by the absorbent core or by the ADL, the amount of the urease inhibitor may be higher in the crotch core region than in the back region of the absorbent core. The amount of the urease inhibitor may be at least 10 weight-%, or at least 20 weight-%, or at least 50 weight-%, or at least 100 weight-% higher in the crotch core region than in the back region. The amount of the urease inhibitor may be not more than 300 weight-% higher in the crotch core region than in the back region.

If the urease inhibitor is comprised by the absorbent core or by the ADL (see below), the amount of the urease inhibitor may be higher in the front region than in the back region of the absorbent core. The amount of the urease inhibitor may be at least 10 weight-%, or at least 20 weight-%, or at least 50 weight-%, or at least 100 weight-% higher in the front region than in the back region. The amount of the urease inhibitor may be not more than 15 weight-%, or not more than 10 weight-% higher in the front region than in the back region.

If the urease inhibitor is comprised by the absorbent core or by the ADL (see below), the amount of the urease inhibitor may be higher in the crotch core region than in the front region of the absorbent core. The amount of the urease inhibitor may be at least 3 weight-%, or at least 5 weight-%, or at least 8 weight-%, or at least 10 weight-% higher in the crotch core region than in the front. The amount of the urease inhibitor may be not more than 25 weight-%, or not more than 20 weight-% higher in the crotch core region than in the front region.

By using the urease inhibitor with the non-homogeneous distribution across the longitudinal dimension as described in the previous paragraphs, higher amounts of urease inhibitor are provided in those areas of the absorbent article, where significant amounts of urine are exposed and stored, namely the crotch region (and crotch core region), and to a slightly smaller extent, in the front waist region and the front region of the absorbent core. Thus, the urine gets readily exposed to the urease inhibitor provided in the absorbent article. At the same time, smaller amounts of urease inhibitor can be applied in areas, specifically in the back waist region and the back region of the absorbent core, reducing the overall use of the urease inhibitor in the absorbent article while still achieving the objective of maintaining skin pH in a desirable range. This enables reduced cost for the urease inhibitor and avoids excessive amounts of the urease inhibitor, given these compounds, though beneficial, should not be applied without a reasonable purpose.

The non-homogeneous distribution as described above can be obtained, e.g., by applying the urease inhibitor on a compound of the absorbent core, such as SAP particles or airfelt (pulp), and/or by applying the urease inhibitor on a compound of one or more layers of the optional ADL. These compounds are often not distributed homogeneously across the longitudinal dimension of the absorbent core and of the absorbent article. Rather, higher amounts are provided in the crotch region and crotch core region, slightly smaller amounts are provided in the front waist region and front region of the absorbent core, and even smaller amounts are provided in the back waist region and back region of the absorbent core. Hence, by not directly applying the urease inhibitor as such in the absorbent article / absorbent core, but indirectly applying it via application on compounds of the absorbent core and/or ADL and subsequent provision of these compounds in the absorbent article, the desired non-homogeneous distribution can be easily obtained. Moreover, given that the absolute amount of the urease inhibitor will generally be rather small, incorporating the urease inhibitor in the absorbent article via other compounds, enables a reliable provision of the urease inhibitor. It may be challenging to obtain the desired distribution in a reliable manner when applying the urease inhibitor, typically in solid form, e.g., as powder, directly as such on a very fast running absorbent article manufacturing line.

It is known that under anaerobic conditions certain bacteria use the denitrification process to generate energy. Specifically, they use nitrates (e.g., derived from ammonia) to oxidate glucose as their metabolic pathway for energy generation. Hence, it has been found that, to reduce ammonia production in an absorbent article during use, having an absorbent structure with high permeability to promote aerobic conditions inside the absorbent article can be beneficial in addition to the use of the urease inhibitor. In one aspect of the present invention, the absorbent core thus has a permeability of from 10-6 cm2 to 10-4 cm2 according to the IPRP test method set out herein. Good permeability can e.g., be achieved by providing the absorbent core with regions of reduced caliper, such as elongated "channels" within the absorbent core, where little or no absorbent material is provided.

During use of an absorbent article, a part of the urine which has been transferred through the topsheet into the absorbent article, may penetrate back through the topsheet before it can be absorbed and stored by the absorbent core. This is also known as "rewet". However, if a urine inhibitor is applied underneath the topsheet, at least a part of the inhibitor may be "washed out" of the absorbent article by being dissolved in the urine which then penetrates back through the topsheet onto the body-facing surface of the absorbent article. To reduce to amount of urease inhibitor which may be transported onto the wearer-facing surface of the absorbent article during use, it is desirable that the absorbent article has good rewet properties. Thus, the absorbent article may have a rewet of less than 150 mg, or less than 120 mg according to the Post Acquisition Collagen Rewet Test Method set out herein.

Urease inhibitor

Urease inhibitors are well known in the art. They may be chemically synthesized or may be derived from natural sources. Some of the known urease inhibitors are toxic. For use in the present invention, the urease inhibitor should not be toxic when used close to the skin of a wearer.

Urease inhibitors include transition metal ions and their soluble salts, such as silver, copper, zinc, ferric, and aluminum salts. The anion may also provide urease inhibition, such as borate, phytate, etc. Compounds of potential value include, but are not limited to, silver chlorate, silver nitrate, mercury acetate, mercury chloride, mercury nitrate, copper metaborate, copper bromate, copper bromide, copper chloride, copper dichromate, copper nitrate, copper salicylate, copper sulfate, zinc acetate, zinc borate, zinc phytate, zinc bromate, zinc bromide, zinc chlorate, zinc chloride, zinc sulfate, cadmium acetate, cadmium borate, cadmium bromide, cadmium chlorate, cadmium chloride, cadmium formate, cadmium iodate, cadmium iodide, cadmium permanganate, cadmium nitrate, cadmium sulfate, and gold chloride.

Other salts that have been disclosed as having urease inhibition properties include ferric and aluminum salts, especially the nitrates, and bismuth salts. Other urease inhibitors include hydroxamic acid and its derivatives; thiourea; hydroxylamine; salts of phytic acid; phenyl phosphoro diamidate/diamino phosphoric acid phenyl ester; metal aryl phosphoramidate complexes, including substituted phosphorodiamidate compounds; phosphoramidates without substitution on the nitrogen.

For the present invention, natural urease inhibitors are preferred. "Natural urease inhibitors", as used herein, are derived from natural sources. The natural urease inhibitor may be plant based, i.e. the urease inhibitor may be a plant having urease inhibiting activity, or may be an extract that has been obtained from such plant.

Natural urease inhibitor for use in the present invention may be green tea (Camellia sinensis), grape seed, red grape seed extract, Aloe vera, Witch hazel (Hamamelis virginiana), Chamomile (Chamomilla recutita), Calendula (Calendula officinalis), Sage (Salvia officinalis), Yucca schidigera, and combinations and mixtures thereof. Further useful urease inhibitors isolated and identified from plants are terpenoids, phenolic compounds, alkaloids, and combinations and mixtures thereof, as well as combinations and mixtures with the urease inhibitors described in the previous paragraph.

Preferred urease inhibitors of the present invention are green tea (Camellia sinensis), grape seed extract, and Epigallocatechin gallate (EGCG), and combinations and mixtures thereof.

EGCG is the ester of epigallocatechin and gallic acid and is a type of catechin. It is found in high content in the dried leaves of green tea (about 7380 mg per 100 g), white tea (about 4245 mg per 100 g), and in smaller quantities, black tea (about 936 mg per 100 g). Hence, for the present invention, it is considered as an extract of (green) tea.

Absorbent articles

The disclosure of absorbent articles herein below is intended to refer to the aspects of the present invention, which refer to the absorbent article as such, as well as to the use of an urease inhibitor in an absorbent article, i.e. the disclosure is meant to describe aspects of the respective absorbent article. While the urease inhibitor is not specifically described in the following disclosure of absorbent articles and the urease inhibitor is also not shown in the Figures, it goes without saying that the absorbent articles of the present invention describe the urease inhibitor as described and claimed herein and in the Examples below.

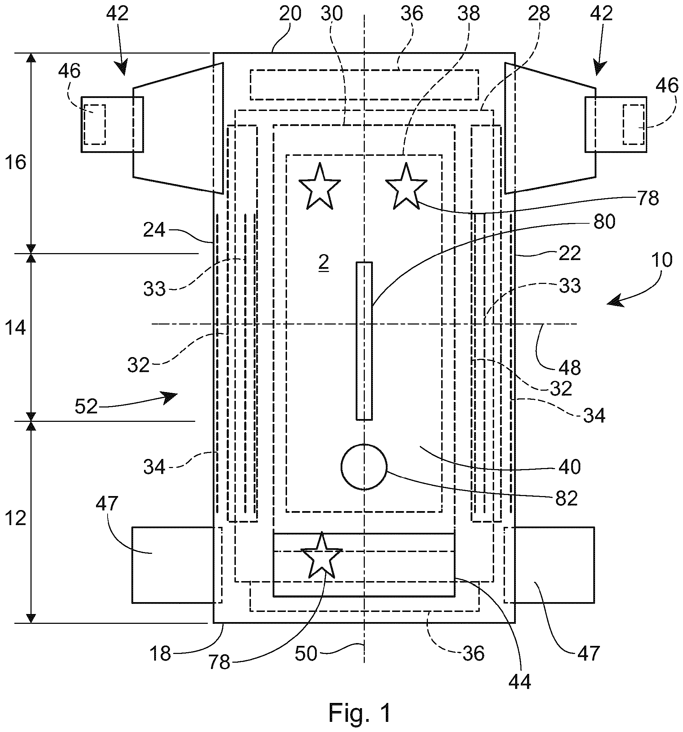

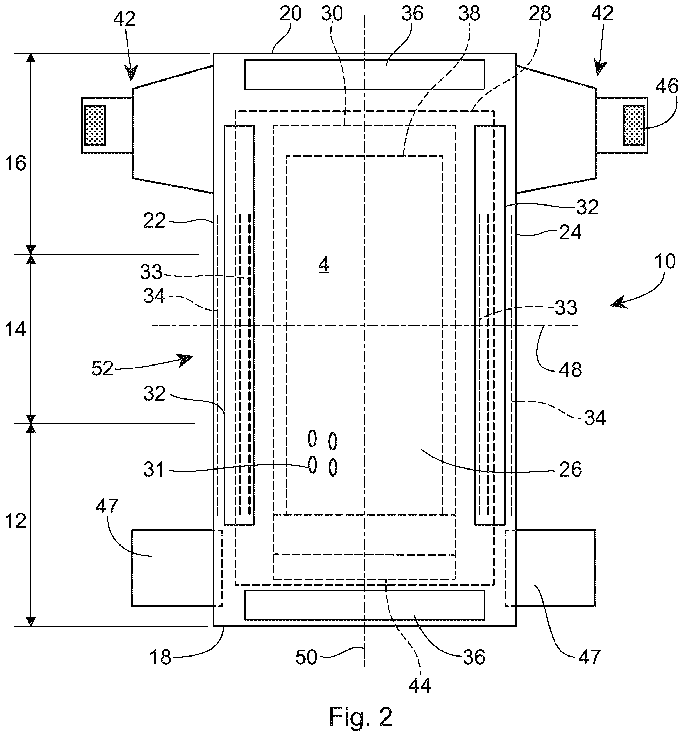

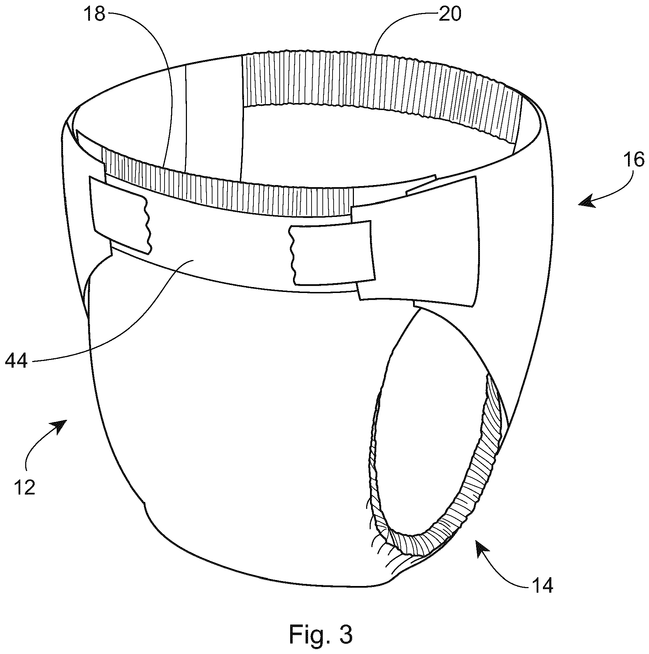

An example absorbent article 10 according to the present disclosure, shown in the form of a diaper (applied on the wearer with tapes), is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, body-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the absorbent article 10. The absorbent article 10 may comprise a front waist edge 18, a back waist edge 20 opposite to the front waist edge 18. The absorbent article 10 further comprise longitudinally extending, transversely opposed side edges 22 and 24 defined by the chassis 52.

The absorbent article 10 comprises a liquid permeable topsheet 26, a liquid impermeable backsheet 28, and an absorbent core 30 positioned between the topsheet 26 and the backsheet 28. Herein below, the term only the terms "topsheet" and "backsheet" is used to describe a liquid permeable topsheet and a liquid impermeable backsheet.

The absorbent article 10 may also comprise one or more pairs of barrier leg cuffs 32 with or without elastics 33, one or more pairs of leg elastics 34, one or more elastic waistbands 36,

The absorbent article may also comprise at least one acquisition and distribution layer (ADL) 38 which is provided in between the topsheet and the absorbent core.

An outer cover material 40, such as a nonwoven material, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a transverse centerline 48 and a longitudinal centerline 50. The transverse centerline 48 extends perpendicular to the longitudinal centerline 50.

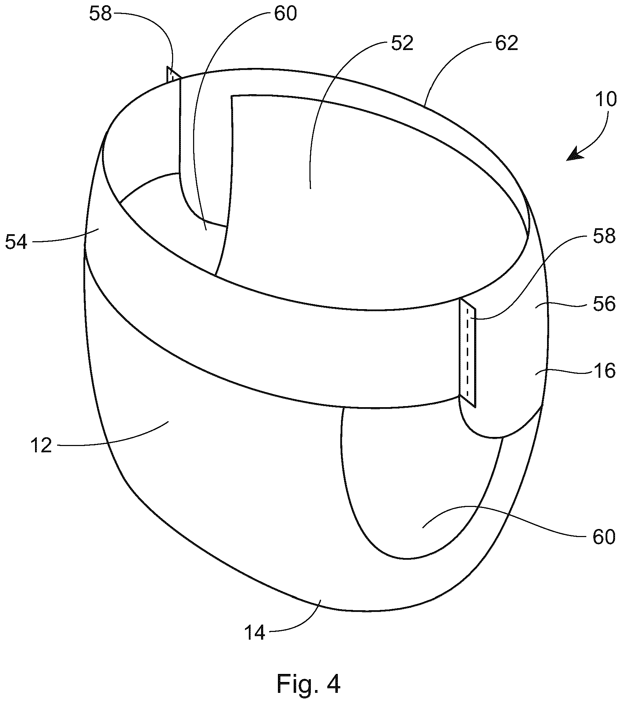

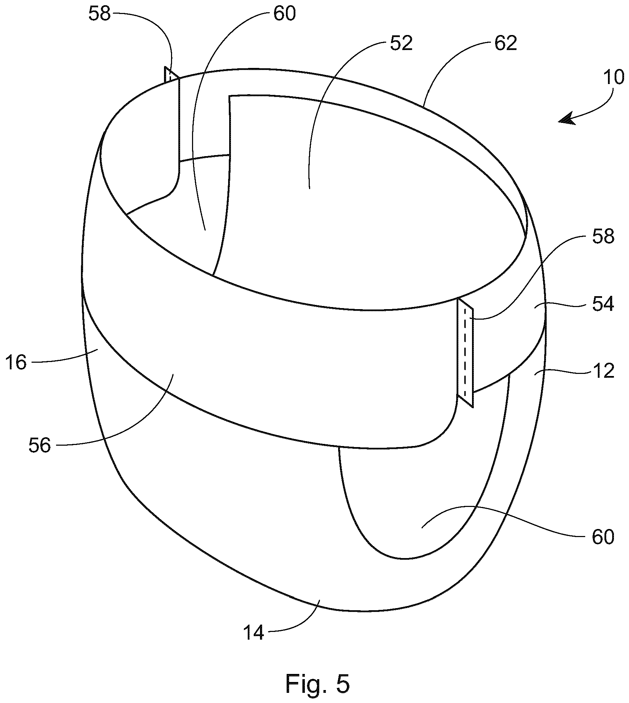

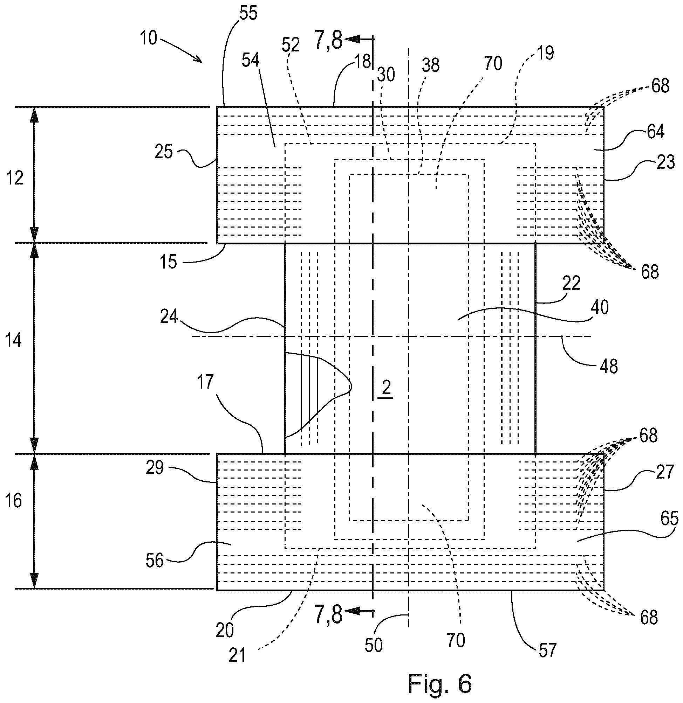

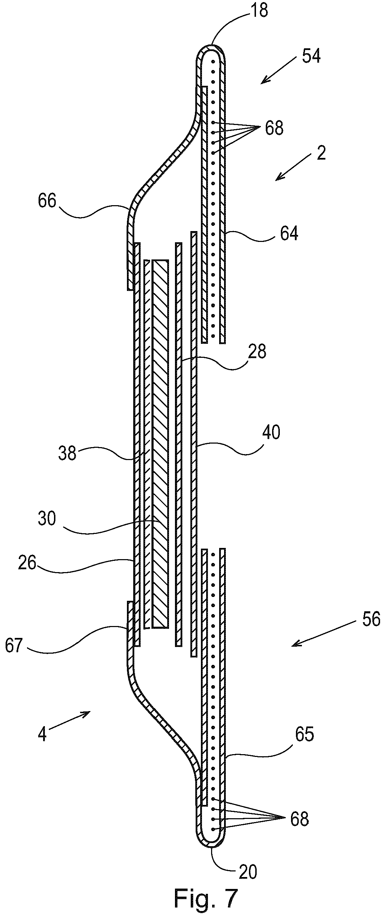

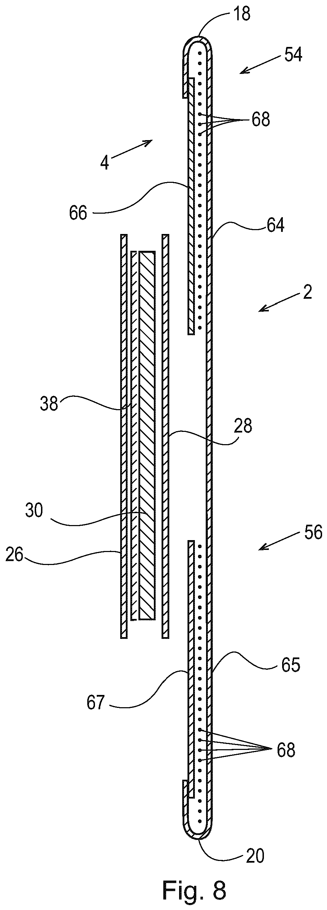

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137. Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 are the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 comprises a topsheet 26, a backsheet 28, and an absorbent core 30 disposed at least partially intermediate the topsheet 26 and the backsheet 28, and optionally at least one acquisition and distribution layer (ADL) 38 which is provided in between the topsheet and the absorbent core, similar to that as described above with respect to Figs. 1-3. The absorbent article 10 may comprise a front belt 54 in the front waist region 12 and a back belt 56 in the back waist region 16. The central chassis 52 (comprising the topsheet, backsheet, the absorbent core and the optional ADL) may be joined to a body-facing surface 4 of the front and back belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

In another form, the absorbent article may be an absorbent insert for use with a reusable outer cover. The insert may be disposable or reusable. The reusable outer cover may comprise a woven or other material and may be configured as a pant or a diaper. In the diaper context, the reusable outer cover may comprise a fastening system used to join a front waist region of the reusable outer cover to a back waist region. The fastening system may comprise snaps, buttons, and/or hooks and loops, for example. The insert may comprise a liquid permeable topsheet, a liquid impermeable backsheet, and an absorbent core positioned at least partially intermediate the topsheet and the backsheet. At least one acquisition and distribution layer (ADL) 38 which is provided in between the topsheet and the absorbent core. The insert may comprise one or more pairs of leg cuffs and may be free of ears, side panels, and/or waistbands. In some instances, a nonwoven material may be positioned on a garment-facing side of the backsheet. A garment-facing surface of the insert may be attached to a body-facing surface of the reusable outer cover via adhesives, hook and loop fasteners, or other methods of joinder. An example insert and reusable outer cover system is disclosed in U.S. Patent No. 9,011,402, issued on April 21, 2015, to Roe et al. The insert or the reusable outer cover may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. Appl. Pub. No. 2013/0211363.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal centerline 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

Topsheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet is liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31), may have any suitable three-dimensional features, and/or may have a plurality of embossments (e.g., a bond pattern). The topsheet may be hydrophilic or hydrophobic or may have hydrophilic and/or hydrophobic portions or layers. If the topsheet is hydrophobic, typically apertures will be present so that bodily exudates may pass through the topsheet.

The topsheet may have an opacity of at least 15 %, or at least 18 %, or at least 20%, or at least 25% as determined according to the opacity test method set out below. Especially if the urease inhibitor used in the absorbent article of the present invention, is neither white nor transparent, applying a topsheet with a good degree of opacity, such as at least 15 %, helps to reduce the visibility of the urease inhibitor through the topsheet. In fact, as the urease inhibitor of the present invention is applied inside the absorbent article, i.e., neither on the body-facing surface of the topsheet nor on the garment-facing surface of the backsheet, urease inhibitors having a certain non-white color can be used. If the urease inhibitor were used on the body-facing surface of the topsheet, body-facing surface of the topsheet would provide a stained and unsightly appearance due to the presence of the urease inhibitor.

The topsheet of the absorbent article of the present invention may not comprise lotion.

Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material.

Absorbent Core

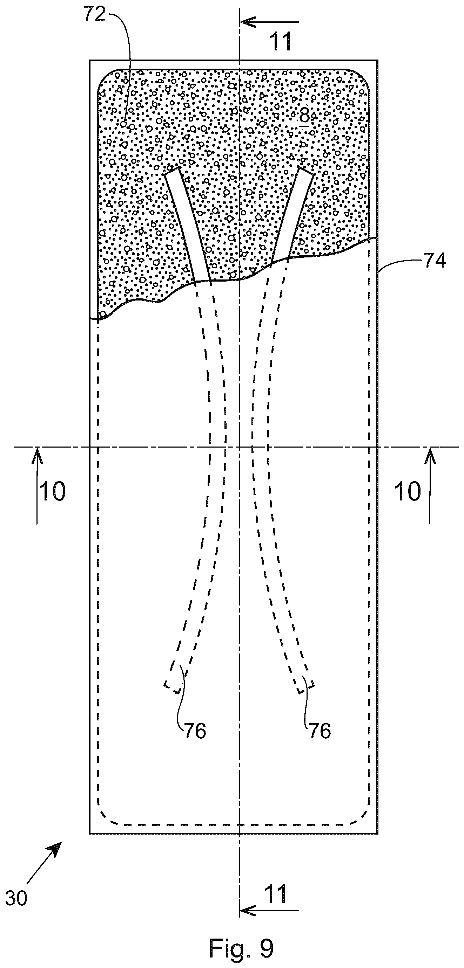

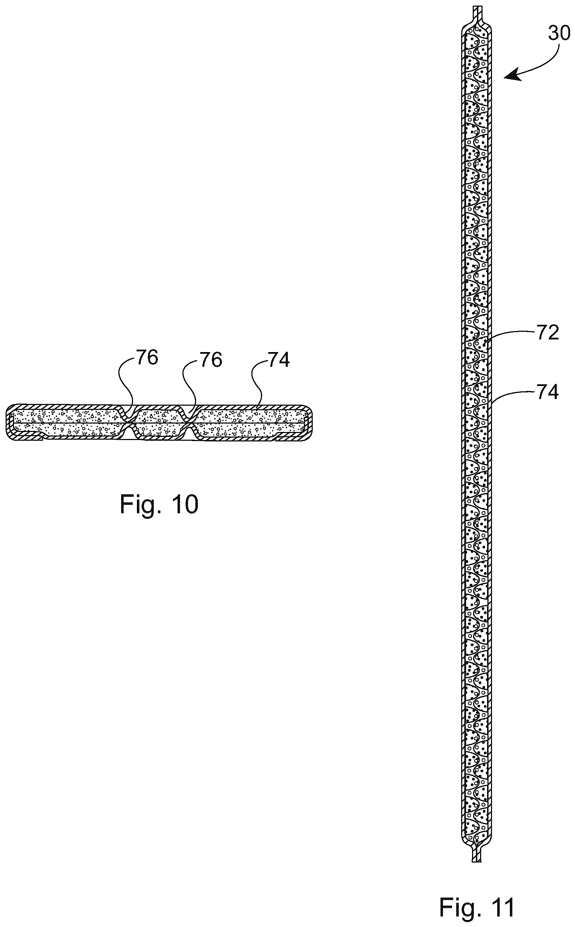

As used herein, the term "absorbent core" 30 refers to the component of the absorbent article 10 intended to store the liquid that enters the absorbent article during use (thus generally having the most absorbent capacity) and that comprises an absorbent material. Referring to Figs. 9-11, in some instances, absorbent material 72 may be positioned within a core bag or a core wrap 74 (the core bag or core wrap being comprised by the absorbent core). The absorbent material may be profiled or not profiled, especially along the longitudinal centerline, depending on the specific absorbent article. "Profiled" means that the absorbent material is not homogeneously distributed across the surface area of the absorbent core. The absorbent core 30 may comprise, consist essentially of, or consist of, a core wrap, absorbent material 72, and glue enclosed within the core wrap. The absorbent material may comprise or consist of a) superabsorbent polymer particles and/or superabsorbent fibers, or b) a mixture of superabsorbent polymer particles and air felt, or c) only air felt, or d) a high internal phase emulsion foam, or e) combinations of any of a) to d). In some instances, the absorbent material may comprise at least 80%, at least 85%, at least 90%, at least 95%, at least 99%, or up to 100% superabsorbent polymers, by weight of the absorbent material. In such instances, the absorbent material may be free of air felt, or at least mostly free of air felt. The absorbent core periphery, which may be the periphery of the core wrap, may define any suitable shape, such as rectangular "T," "Y," "hour-glass," or "dog-bone" shaped, for example. Preferably, the absorbent core has a rectangular shape. An absorbent core periphery having a generally "dog bone" or "hour-glass" shape may taper along its width towards the crotch region 14 of the absorbent article 10.
Referring to Figs. 9-11, the absorbent core 30 may have areas with reduced caliper (wherein the term "reduced" includes areas with no caliper, i.e. areas free of the material of the absorbent core). Areas with reduced caliper may be areas having little or no absorbent material 72, where a body-facing surface of the core bag 74 may be joined to a garment-facing surface of the core bag 74. These areas having little or no absorbent material may be referred to as "channels" 76. These channels can embody any suitable shapes and any suitable number of channels may be provided. In other instances, though less preferred, the absorbent core may be embossed to create the impression of channels. The absorbent core in Figs. 9-11 is merely an example absorbent core. Many other absorbent cores with or without channels are also within the scope of the present disclosure.

Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a body-facing surface 4 of the absorbent article 10 and provide improved containment of body exudates approximately at the junction of the torso and legs of the wearer. The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front waist edge 18 and the back waist edge 20 of the absorbent article 10 on opposite sides of the central longitudinal centerline 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front waist edge 18 and the back waist edge 20. The leg elastics 34 essentially cause portions of the absorbent article 10 proximate to the chassis side edges 22, 24 to help form a seal around the legs of the wearer. The leg elastics 34 may extend at least within the crotch region 14.

Elastic Waistband

Referring to Figs. 1 and 2, the absorbent article 10 may comprise one or more elastic waistbands 36. The elastic waistbands 36 may be positioned on the garment-facing surface 2 or the body-facing surface 4. As an example, a first elastic waistband 36 may be present in the front waist region 12 near the front belt waist edge 18 and a second elastic waistband 36 may be present in the back waist region 16 near the back waist edge 20. The elastic waistbands 36 may aid in sealing the absorbent article 10 around a waist of a wearer and at least inhibiting bodily exudates from escaping the absorbent article 10 through the waist opening circumference. In some instances, an elastic waistband may fully surround the waist opening circumference of an absorbent article.

Acquisition Materials

Referring to Figs. 1, 2, 7, and 8, at least one acquisition and distribution layer (ADL) 38 may be provided between the topsheet 26 and the absorbent core 30 may be present at least partially intermediate the topsheet 26 and the absorbent core 30. The ADL comprises acquisition materials which are typically hydrophilic materials that provide significant wicking of bodily exudates. These materials may dewater the topsheet 26 and quickly move bodily exudates into the absorbent core 30. The acquisition materials of the ADL may comprise one or more nonwoven materials, foams, cellulosic materials, cross-linked cellulosic materials, air laid cellulosic nonwoven materials, spunlace materials, or combinations thereof, for example. Typically, the one or more layers of the ADL may each have a width and length that are smaller than the width and length of the topsheet 26. The ADL may have one or more areas with reduced caliper ((wherein the term "reduced" includes areas with no caliper, i.e. areas free of the material of one, more than one, or all layers of the ADL), such as channels, as described above with reference to the absorbent core 30 (including the embossed version). The channels in the ADL may align or not align with channels in the absorbent core 30. In an example, a first layer of the ADL may comprise a nonwoven material and as second layer of the ADL may comprise a cross-linked cellulosic material. The second layer of the ADL may be provided between the first layer of the ADL and the absorbent core. The first layer of the ADL may be provided between the topsheet and the second layer of the ADL.

Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or vice versa.

Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. The ears, or portions thereof, may be elastic or may have elastic panels. In an example, an elastic film or elastic strands may be positioned intermediate a first nonwoven material and a second nonwoven material. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a body-facing surface 4, on the garment-facing surface 2, or intermediate the two surfaces 4, 2.

Masking Layer

One or more masking layers or materials may be provided in the absorbent articles 10. A masking layer may be a layer that provides a cushiony feel when the absorbent article is touched from the garment-facing surface 2 or the body-facing surface 4. The masking layer may "mask" a grainy feel potentially caused by the absorbent material 72, such as superabsorbent polymers. The masking layer may "mask" bodily exudates from being visible when viewing the body-facing surface 4 or the garment-facing surface 2 of the absorbent article 10. The masking layer may also mask the visibility of the urease inhibitor. The masking layer may have a basis weight in the range of about 15 $g/m^2$ to about 50 $g/m^2$ or about 15 $g/m^2$ to about 40 $g/m^2$. The masking layer may comprise one or more nonwoven materials (e.g., a hydroentangled nonwoven material), foams, pulp layers, and/or other suitable materials. The masking layer may be the outer cover material 40. The masking layer may be the layer forming the garment-facing side or the body-facing side of the core bag 74. The masking layer may be a separate material positioned intermediate the garment-facing side of the core bag 75 and the liquid impermeable backsheet 28.

Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Arrays

"Array" means a display of packages comprising disposable absorbent articles of different article constructions (e.g., different elastomeric materials [compositionally and/or structurally] in the side panels, side flaps and/or belts flaps, different graphic elements, different product structures, fasteners or lack thereof). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

Sanitary Napkin

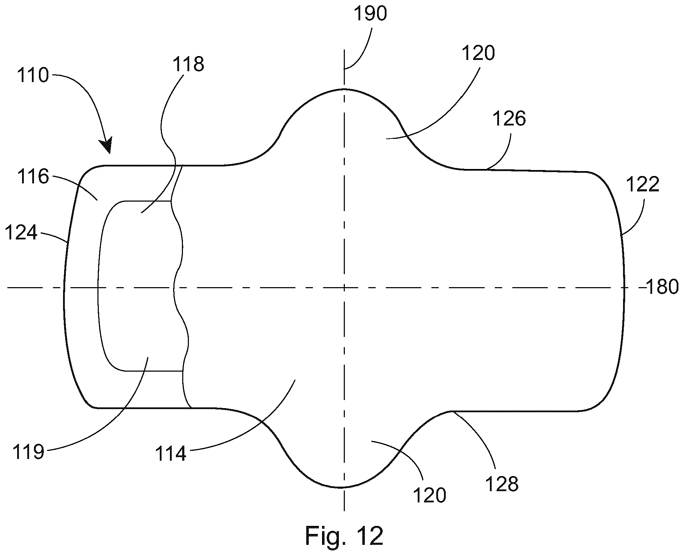

Referring to Fig. 12, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the ADL disclosed above. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal centerline 180 of the sanitary napkin 110. The sanitary napkin 110 may also comprise a transverse centerline 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal centerline 180 may extend from a midpoint of the front edge 122 to a midpoint of the back edge 124. The transverse centerline 190 may extend from a midpoint of the first side edge 128 to a midpoint of the second side edge 128. The sanitary napkin 110 may also be provided with additional features commonly found in sanitary napkins as is known in the art.

Examples Cross-sections of Absorbent Articles

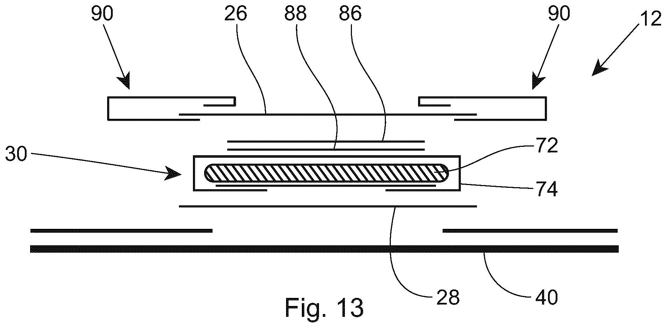

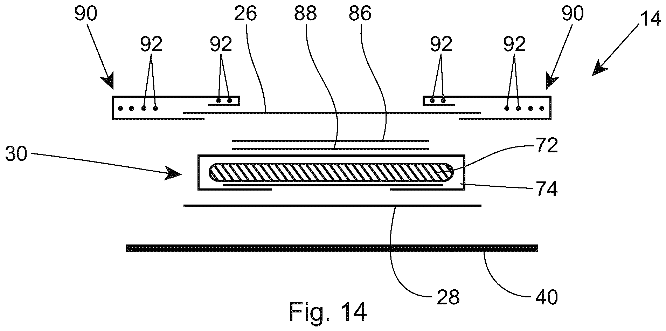

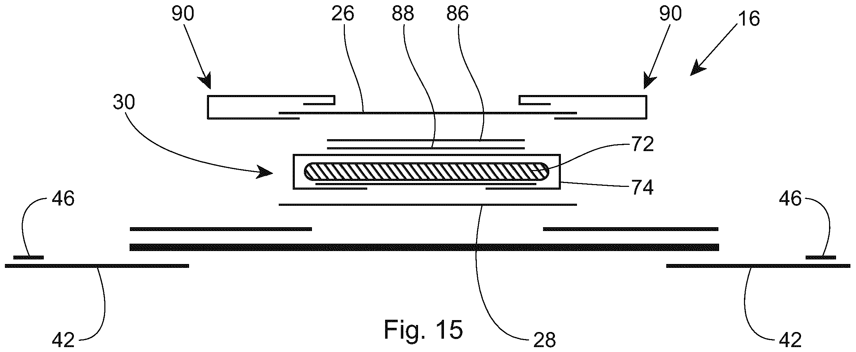

Figs. 13-15 illustrate example cross-sectional views of absorbent articles within the scope of the present disclosure. Fig. 13 is an example cross-sectional view taken within a front waist region 12 of an absorbent article. Fig. 14 is an example cross-sectional view taken within a crotch region 14 of an absorbent article. Fig. 15 is an example cross-sectional view taken within a back waist region 16 of an absorbent article. In Figs. 13-15, an outer cover material is element 40, a topsheet is element 26, a backsheet is element 28, an absorbent core is element 30, with the core bag being element 74, an absorbent material is element 72, and an ADL is element 86. The ADL 86 may comprise cross-linked cellulosic material and is optional. An acquisition material is element 88. Barrier leg cuffs are elements 90. Elastics in the barrier leg cuffs are elements 92. Back ears are elements 42. Fasteners on the back ears 42 are elements 46. Construction glues and/or bonds between the various layers and/or components have been removed for clarity. Other cross-sectional configurations known to those of skill in the art are also within the scope of the present disclosure.

Bio-Based Content for Components

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260TM, SHE150TM, or SGM9450FTM (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Recycle Friendly and Bio-Based Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. Appl. Publ. No. 2019/0192723, published on June 27, 2019.

**Test methods**

**Urease Activity Test Method**

The Urease Activity Test Method measures urease activity in absorbent articles after treatment with a test solution containing urease and synthetic urine containing urea. In this method, like test samples, excised from like absorbent articles of interest, are placed in test containers and treated with the test solution containing urease and synthetic urine containing urea. A layer of collagen is also present in each test container. Each test container is closed and incubated at 37°C for approximately 4 hours. Thereafter, urease activity is characterized by measuring (a) gas-phase ammonia release, (b) pH of the top-most layer of the absorbent article sample (i.e., the body-facing layer of the absorbent article) and (c) pH of collagen.

Sample and Material Preparation

For each absorbent article of interest, test samples are excised from each of six like absorbent articles of interest. From each like absorbent article of interest, the excised test sample is a circular disk 10.0 cm in diameter and centered at a point lying on the transverse centerline that is a distance rearward of the front edge of the absorbent core of the absorbent article that is 48.5% of the entire length of the absorbent core (i.e., in longitudinal dimension). That is, the point at which each test sample is centered is along the transverse centerline and slightly forward of the intersection of the absorbent core's transverse and longitudinal centerlines. The disk is cut or punched through all layers present in the absorbent article at this location, and the test sample subsequently maintains the internal organization of structure of layers as was present in the intact absorbent article.

Cylindrical, screw-top, polypropylene containers, approximately 500 mL in volume and measuring approximately 10.5 cm in diameter and 8 cm in height, are used to house each test sample during and after addition of test solution. The bottom of the cylindrical containers is large enough in diameter such that a test sample can lie flat. In each lid of the test container is drilled a centered hole 8 mm in diameter. This hole is immediately sealed over with tape and is opened only after incubation of the container at 37 °C.

For each screw-top container to be used during analysis, an annulus (made of acrylic or polycarbonate) is fashioned with an outer diameter of 7.5 cm, an inner diameter of 5 cm, and a thickness of 7.5 mm.

Circular collagen disks, 7.0 cm in diameter, to be positioned individually above each test sample, are cut from commercially available collagen sheets (Naturin COFFI clear, having a basis weight of $33.5 \pm 0.4$ g/m$^2$, Viscofan Group, Tajonar, Spain) or equivalent. Each circular collagen disk is placed on top of an annulus. This assembly allows positioning of the collagen disks above each test sample, such that there is no direct contact between the collagen disk and the test sample.

Synthetic Urine is prepared by dissolving 2% w/w urea, 0.9% w/w sodium chloride, 0.11% w/w magnesium sulfate monohydrate, 0.079% w/w calcium chloride monohydrate in deionized water.

Urease stock solution (23.5 U/ml) is made by dissolving Jack Bean Urease (Catalog number U1500, Millipore Sigma, St. Louis, MO, USA, or equivalent) in deionized water.

For each sample to be tested, 75 ml of the test solution is prepared immediately before use by mixing 67.5 ml of Synthetic urine and 7.5 ml of urease stock solution at ambient lab temperature ($23\pm1$ °C).

Test Procedure

Each test sample is placed in the above-mentioned cylindrical screw-top container with its skin-facing side facing upward. 75 mL of test solution (prepared just before use) is then poured over the test sample. Within one minute, after visually confirming that the test solution is fully absorbed by the test sample and no free liquid is present above the test sample, the annulus bearing a single layer of collagen is placed above the test sample such that there is no direct contact between the collagen disk and the test sample. In the event that free test solution remains visible in the test container after one minute, the free test solution is removed using appropriate means such as a pipette and the annulus bearing a layer of collagen placed above the test sample. A container lid is immediately used to close the test container, and the test container is incubated at 37 °C for $4.0 \pm 0.25$ hours in a climate-controlled oven/incubator.

Measurement and Reporting

Immediately following the incubation period, the test container for each test sample being characterized is removed from the oven/incubator and measured.

For each test container and test sample, the concentration of gas-phase ammonia in the headspace in the test container above the test sample is measured using the Draeger flow-tube-based gas-phase ammonia detection system (for example, that available from Draegerwerk AG & Co. KGaA, Luebeck, Germany). Suitable apparatus for this particular application are Draeger short-term detection tubes - Ammonia 5/b, catalog no. 81 01 941 and Draeger Accuro Pump, catalog no. 64 00 000, or equivalent. The tape sealing the orifice in the lid of the test container is removed and the ammonia tube is inserted to a depth of $3\pm0.5$ cm into the test container. The Draeger pump is used to withdraw a sample of the gas-phase in the test container as per the manufacturer's instructions, drawing 100 ml two times (i.e, 200 ml) and then divide the level of color change by 2 to obtain the ppm value for ammonia. Depending on the concentration of headspace ammonia, it may be necessary to use alternative short term ammonia detection tubes, suited to the concentration being measured. The level of colour change in the ammonia tube is used to determine the amount of ammonia (ppm) in the gas-phase of the container. For each test container and test sample, the gas-phase ammonia concentration is recorded to the nearest 1 ppm.

Following the measurement of gas-phase head-space ammonia (approximately 30-60 minutes), for each test container and test sample, the lid of each test container is unscrewed and removed. The collagen disk and annulus are withdrawn/extracted from the test container so as to avoid contact between the collagen to the sides of the container. The pH of the collagen layer is measured using a skin and scalp pH meter (catalog number HI981037, Hanna Instruments, Smith-field, RI, USA, or equivalent). In general, the collagen will have developed some tack during the incubation and adheres to the annulus, thereby supporting modest pressure against the collagen surface. This allows collagen to be supported on the ring when the pH probe is in contact to the collagen disk for pH measurement. The pH meter is calibrated before measurements with buffer solutions of pH 4 and 7 per the instructions of the manufacturer. The pH of the collagen associated with each test sample is recorded to the nearest 0.01 of the test sample collagen pH.

Finally, for each test sample, the pH of the topmost layer of absorbent article test sample is measured using the skin and scalp pH meter. While the pH meter is placed in contact with the topmost layer of the test sample, the entire test sample (all layers) remains present intact underneath the topmost layer. The pH of the topmost layer of each test sample is recorded to the nearest 0.01 of the test sample topsheet pH.

The arithmetic mean of the gas-phase ammonia concentration among all test samples analyzed is calculated and reported to the nearest 0.1 ppm as the gas-phase ammonia concentration of the sample absorbent article. The arithmetic mean of the topsheet pH among all test samples analyzed is calculated and reported to the nearest 0.1 as the topsheet pH of the sample absorbent article.

The arithmetic mean of the collagen pH among all test samples analyzed is calculated and reported to the nearest 0.1 as the collagen pH due to the sample absorbent article.

## Modified Fluid Acquisition Test

The Modified Fluid Acquisition ("MFA") Test is designed to measure the speed at which 0.9 weight-% saline solution is absorbed into an absorbent core that is compressed at 2.07 kPa. Additional layers may be placed on top and/or below the absorbent core for the Modified Fluid Acquisition Test (see Examples below), in which case the MFA determines the speed at which 0.9 weight-% saline solution is absorbed into an absorbent core and the additional layers that is compressed at 2.07 kPa.

A known volume is introduced four times, each successive dose starting five (5) minutes after the previous dose has absorbed. Times needed to absorb each dose are recorded. The test fluid is 0.9 weight-% w/v saline solution and is prepared by weighing 9.0 g $\pm$ 0.05g of NaCl into a weigh boat, transferring it into a 1L volumetric flask, and diluting to volume with de-ionized water.

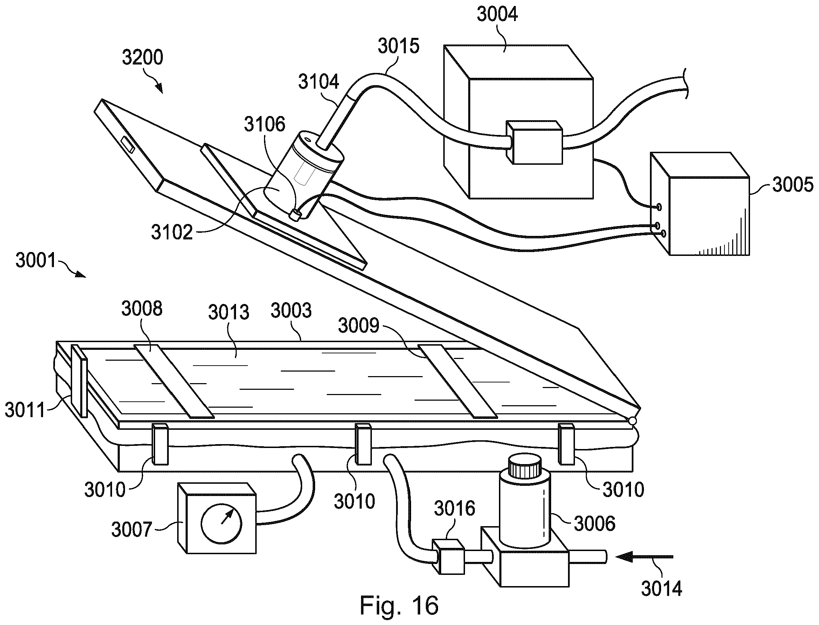

The MFA apparatus is depicted in FIG. 16 through FIG. 18B. The MFA apparatus comprises a bladder assembly 3001 and a top plate assembly 3200 that includes a deposition assembly 3100. A controller 3005 is used to 1) monitor the impedance across electrodes 3106, recording the time interval 0.9 weight-% saline solution is in a cylinder 3102, 2) interface with a liquid pump 3004 to start/stop dispensing, and 3) time intervals between dosing. The controller 3005 is capable of recording time events to $\pm$ 0.01 sec. A house air supply 3014 is connected to a pressure regulator 3006 capable of delivering air at a suitable flow/pressure to maintain 2.07 kPa in the bladder assembly 3001. A liquid pump 3004 (Ismatec MCP-Z gear pump, available from Cole Palmer, Vernon Hills, IL or equivalent) capable of delivering a flow of 10-80 mL at a rate of 3-15 mL/s is attached to a stainless-steel tube 3104 of the deposition assembly 3100 via Tygon® tubing 3015.

The bladder assembly 3001 is constructed of 12.7 mm Plexiglas with an overall dimension of 80 cm long by 30 cm wide by 10 cm tall. A manometer 3007 to measure the pressure inside the assembly and a pressure gauge 3006 to regulate the introduction of air into the assembly are installed through two holes through the light side. A bladder 3013 is assembled by draping a 50 mm by 100 mm piece of silicone film, (thickness 0.02", Shore A durometer value of 20, available as Part# 86435K85 from McMaster-Carr, Cleveland, OH) over the top of the box with enough slack that the film touches the bottom of the box at its center point. An aluminum frame 3003 with a flange is fitted over the top of the film and secured in place using mechanical clamps 3010.

When in place, the assembly should be leak free at a pressure of 3.45 kPa. A front 3008 and back 3009 sample support of 5 cm by 30 cm by 1 mm are used to anchor the sample. The absorbent core (optionally with additional layers on top and/or below) is attached to the top surface of the sample supports by either adhesive tape or mechanical "hook" fasteners. These supports can be adjusted along the length of the aluminum frame 3003 via a simple pin and hole system to accommodate different size absorbent cores and to correctly align their loading point.

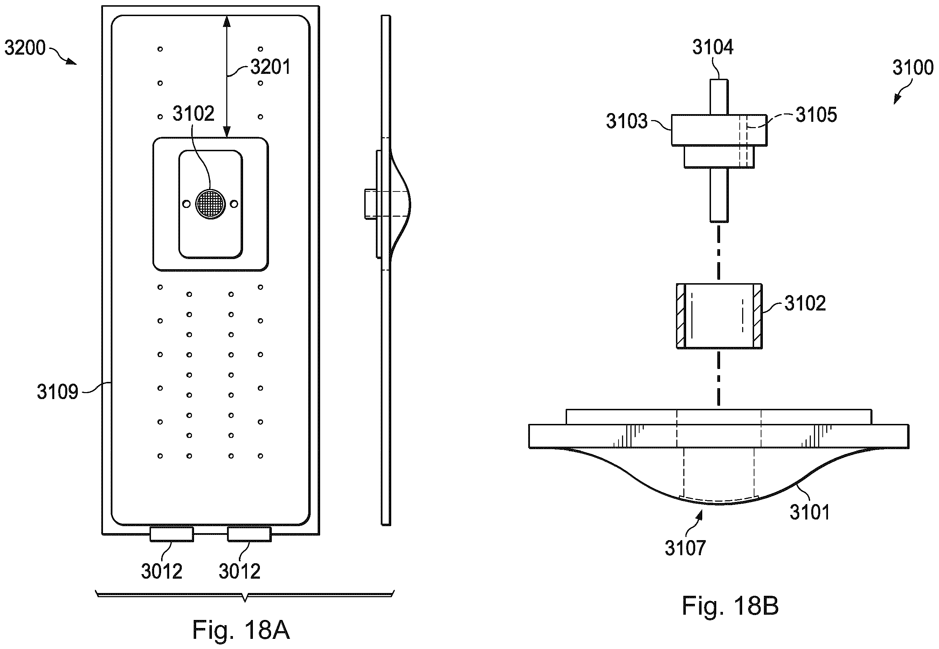

The top plate assembly 3200 is constructed of an 80 cm by 30 cm piece of 12.7 mm Plexiglas reinforced with an aluminum frame 3109 to enhance rigidity. The plate has a cutout l70 mm wide by 201 mm long centered laterally on the plate, l70 mm from the front of the plate 3201 for mounting of the deposition assembly. In addition, the top plate has thirty-six (36) 3.2 mm diameter holes drilled through it distributed as shown in FIG. 18A. The holes prevent air from being trapped under the top plate as the bladder is inflated. The top plate assembly 3200 is connected to the bladder assembly 3001 via two hinges 3012. During use, the top assembly is closed onto the bladder assembly and locked into place using a mechanical clamp 3011.

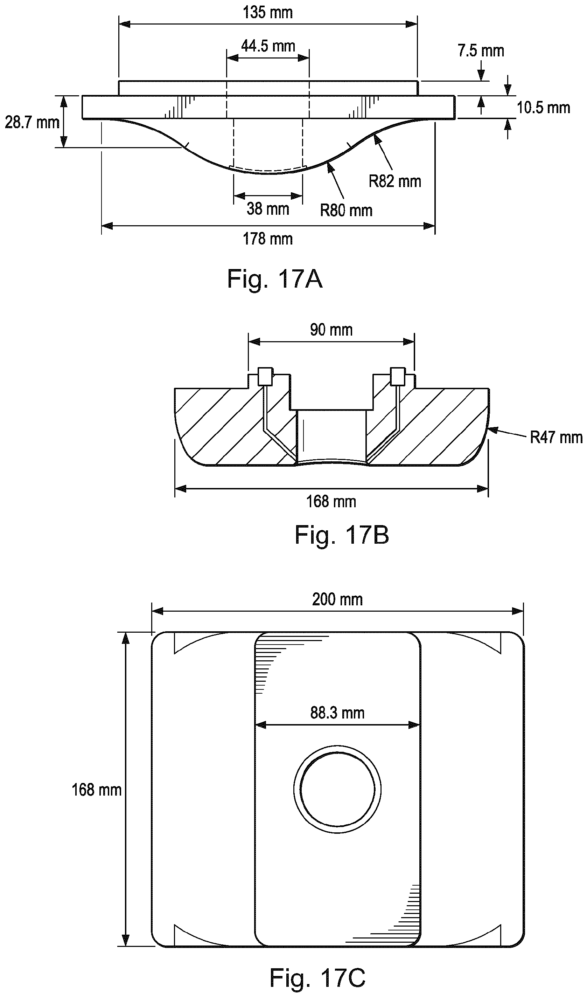

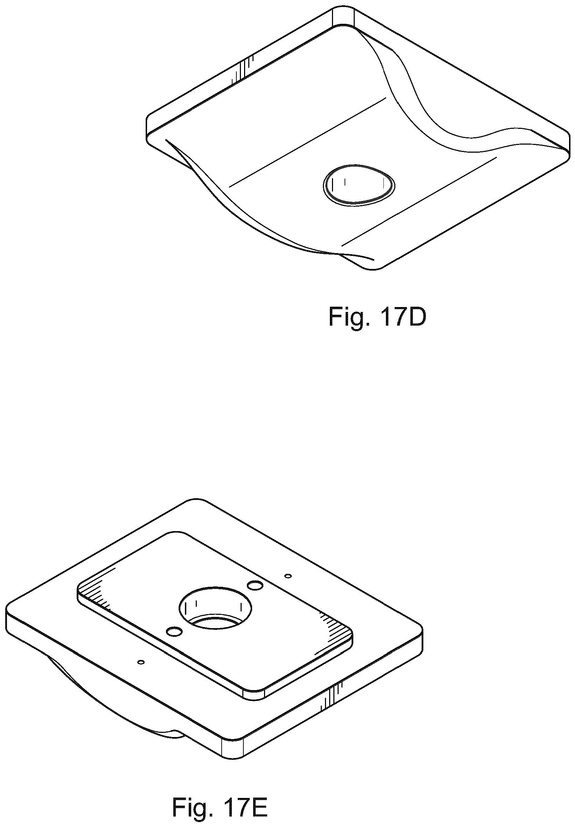

The deposition assembly 3100 is fitted into the top plate 3200 and includes 1) a liquid introduction cylinder 3102, 2) a curved surface 3101 at the loading point of the absorbent core and 3) electrodes 3106 that are used to detect fluid in the cylinder 3102. The detailed dimensions of the curved component are provided in FIG. 17A to FIG. 17E. FIG. 17A is a side view of the curved component. FIG. 17B is an end view of the curved component. FIG. 17C is a bottom view of the curved component. FIG. 17D is a bottom perspective view of the curved component. FIG. 17E is a top perspective view of the curved component. This curved component can be milled or 3D printed. The top portion of the introduction cylinder is a 50.8 mm O.D. Plexiglas cylinder 3102 with a 38.1 mm I.D. This is fitted into the curved component to give the introduction cylinder a total height of 100 mm. Imbedded electrodes run from connectors on the upper surface of the curved component and terminate flush with an inside wall of the introduction cylinder, 2 mm from the bottom of the cylinder. The two electrodes are positioned 180 degrees apart. A nylon screen 3107 is cut and affixed flush with the bottom of the cylinder such that the sample cannot swell into the cylinder. A 5 mm semi-circle is cut in the screen in the immediate area of the two electrodes. The deposition assembly is inserted into the top plate as shown in FIG. 18A such that the curved surface is flush with the bottom of the top-plate assembly 3200. The introduction cylinder 3102 is topped with a loose-fitting nylon cap 3103. The cap has a 6.35 mm O.D. steel tube 3104 inserted through its center. When the cap is in place, the bottom of the tube ends 20 mm above the screen 3107. The cap also has an air hole 3105 to ensure negative pressure does not impede the absorption speed.

Place the absorbent core flat onto a lab bench and identify the intersection of the longitudinal centerline with the loading point as defined in the following Table.

Conditions for Modified Fluid Acquisition Testing:

| Loading Point from front of Core | Gush Volume | Flow Rate | Delivery Time | Number of subsequent gushes |
|---|---|---|---|---|
| mm | mL | mL/s | s | |
| 170 | 75 | 15 | 5 | 4 |

Attach the end of the absorbent core, which is intended to be placed towards the front end of the absorbent article (i.e., the front waist region for a diaper or pant), to the top surface of the front sample plate 3008 by either adhesive tape or mechanical "hook" fasteners with a topsheet facing upward. For absorbent cores which are symmetric along their transverse axis, it is not relevant which end of the absorbent core is attached to the top surface of the front sample plate 3008. The placement is such that no parts of the absorbent core overlay the plate. The sample plate 3008 is attached to the aluminum frame 3003 such that the size-dependent (i.e., size of the absorbent article into which the absorbent core is intended to be provided) Loading Point (as defined in the Table above) of the absorbent core will be centered longitudinally and laterally within the cylinder 3102 when the top plate assembly has been closed. The end of the absorbent core, which is intended to be placed towards the back end of the absorbent article, is secured to the back sample plate 3009 by either adhesive tape or mechanical "hook" fasteners, once again ensuring that no parts of the absorbent core overlay the plate. The back sample plate 3009 is then attached to the aluminum frame 3003 such that the absorbent core is taunt but not stretched. The top plate assembly is closed and fastened, and the bladder is inflated to 2.07 kPa $\pm$ 0.07 kPa. The pressure is maintained at this level during the complete loading sequence of the test.

The pump 3004 is primed and then calibrated to deliver the size-dependent volume and flow rate selected from the Table above. Volume and flow rate must be within $\pm$ 2% of target. The cap 3103 is placed into the cylinder 3102. The controller 3005 is started, which in turn delivers the first dose of 0.9 weight-% saline solution. After the volume has been absorbed, the controller waits for 5.0 minutes before addition of the next dose. This cycle is repeated for a total of four doses. If the fluid leaks out of or around the article (i.e., is not absorbed into the article) then the test is aborted. Also, if any acquisition time exceeds 1200 seconds, the test is aborted. The acquisition time is defined as the difference between the start time (i.e., when the 0.9 weight-% saline is first introduced into the cylinder and that conducting fluid completes the circuit between the electrodes) and the stop time (i.e., when the fluid has completely drained from the cylinder and the circuit between the electrodes is broken). Acquisition times are recorded by the controller for each dose to the nearest 1.0 second. After the last dose is acquired, pressure is applied for an additional 10 minutes. Open the pressure relief valve 3016 to deflate the bladder and then remove the sample from the acquisition system.

In the same fashion, run a total of four (4) replicates for each absorbent core to be evaluated. Calculate and report the Acquisition Times (sec) for each dose as the arithmetic mean of the replicates to the nearest 1.0 sec.

As said above, additional layers may be placed on top of the absorbent core, prior to testing, such as topsheet materials and/or layers that are used as acquisition materials in an absorbent article. Also, a liquid impervious polyolefin film may be provided underneath the absorbent core, i.e., between the absorbent core and the sample plate.

## Post Acquisition Collagen Rewet Test Method

This method requires a collagen film having a Fixed Height Frit Absorption (FHFA at 0cm) between 0.48 g/g and 0.66 g/g and FHFA at 20cm between 0.15g/g and 0.21 g/g as measured according to the method described below. The collagen film has also a basis weight of 31.5 +/-3.5 g/m$^2$. The collagen film can be purchased from Viscofan Group, 31192 Tajonar-Navarra, Spain, under the designation of Naturin COFFI clear, or equivalent material having the characteristics and basis weight as described above.

Before executing the test, the collagen film as is prepared by being cut into circular sheets of 90 mm (3.54 inches) diameter e.g. by using a sample cutter device, and by equilibrating the film in the controlled environment of the test room (see Modified Fluid Acquisition Test Method) for at least 12 hours (tweezers are to be used for all handling of the collagen film).

At least 5 minutes, but not more than 6 minutes after the last gush, which has been performed in the above Modified Fluid Acquisition Test Method, is absorbed, the cover plate and weights are removed, and the test sample is carefully placed flat on a lab bench.

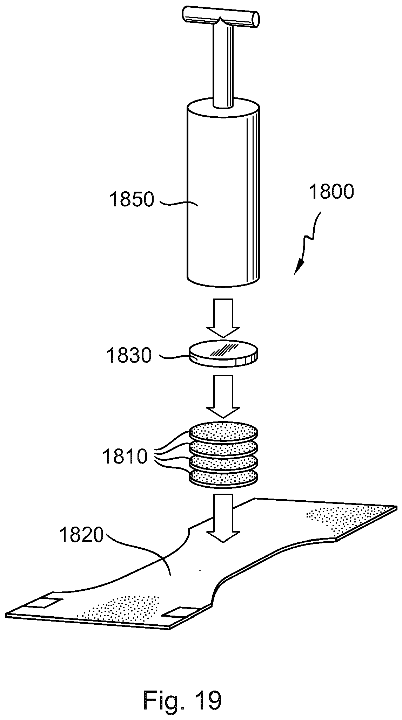

Four sheets of the precut and equilibrated collagen material 1810 are weighed with at least one milligram accuracy, and then positioned centered onto the loading point of the article, as defined in the Modified Fluid Acquisition Test Method, and covered by a plate 1830 made of Poly(methyl methacrylate) (PMMA) (e.g. Perspex$^®$) of 90 mm (3.54 inches) diameter, and about 20 mm (0.78 inches) thickness. A weight (1850) of 15 kg is carefully added (also centered). After 30 +/-2 seconds the weight and Perspex$^®$ plate are carefully removed again, and the collagen films are reweighed (See the system 1800 in Figure 19).

The Rewet result is the moisture pick up of the collagen film, expressed in mg. Four products for each option are tested in this fashion and the average rewet is calculated.

## Fixed Height Frit Absorption (FHFA) at 20 cm and at 0 cm Test Methods

This test is suitable of measuring the uptake of a material under the conditions of suction pressures of 20 cm or of 0 cm of fluid, for example of a saline solution (0.9% wt. NaCl solution) after 30s.

*General apparatus setup*

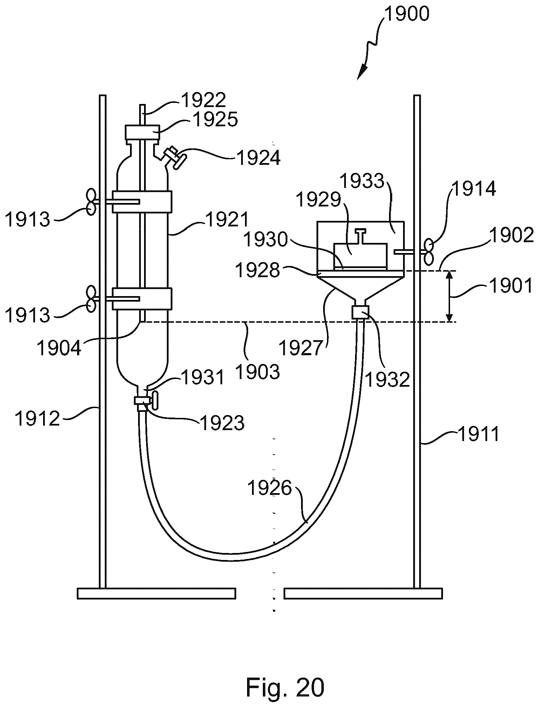

Figure 20 shows the FHFA measurements setup 1900: a suitable fluid delivery reservoir 1921, has an airtight stopcock 1924 to allow the air release during the filling of the equipment. An open-ended glass tube 1922 having an inner diameter of 10 mm extends through a port 1925 in the top of the reservoir such that there is an airtight seal between the outside of the tube and the reservoir, this allows maintaining the required zero level of the hydro head during the experiment regardless the amount of liquid in the reservoir. Reservoir 1921 is provided with delivery tube 1931 having an inlet at the bottom of the reservoir, a stopcock 1923, with the outlet connected to the bottom 1932 of the sample holder funnel 1927 via flexible plastic tubing 1926 (e.g., Tygon$^®$). The Fluid reservoir is firmly held in position by means of standard lab clamps 1913 and a suitable lab support 1912. The internal diameter of the delivery tube 1931, stopcock 1923, and flexible plastic tubing 1926 enables fluid delivery to the sample holder funnel 1927 at a high enough flow rate such that such flowrate is higher than the flowrate absorbed by the collagen sample in the conditions of the experiment and exclude that the measured uptake is limited by the fluid flowrate supplied by the equipment system. The reservoir 1921 has a capacity of approximately 1 liter. Other fluid delivery systems may be employed provided that they are able to deliver the fluid to the sample holder funnel 1927 maintaining the zero level of the hydrostatic liquid pressure 1903 at a constant height during the whole experiment.

The sample holder funnel 1927 has a bottom connector with an internal diameter of 10 mm, a measurement and a chamber 1933 where a glass frit 1928 is accommodated. The sample holder chamber has a suitable size to accommodate the sample 1930 and the confining pressure weight 1929. The frit is sealed to the wall of the chamber 1933. The glass frit has pore of specific size of 16-40μm (glass frit type P 40, as defined by ISO 4793) and a thickness of 7 mm.

The confining pressure weight 1929 is a cylinder with a diameter identical to the sample size (6 cm) and a weight of 593.94 g so to apply exactly 2.06 kPa of confining pressure to the sample 1930. The sample holder funnel 1927 is precisely held in position using a suitable lab support 1911 through a standard lab clamp 1914. The clamp should allow an easy vertical positioning of the sample holder funnel 1927 such that the top of the glass frit 1928 can be positioned at a) the same height (+/- 1 mm) of the bottom end 1904 of the open-ended glass tube 1922 and b) exactly 20 cm (+/- 1 mm) above the bottom end 404 of the open-ended glass tube 1922. Alternatively, two separated clamps are positioned at the abovementioned setups a and b and the sample holder funnel is alternatively moved from one to the other. During the non-usage time, the instrument is kept in proper operating conditions flooding the sample holder funnel 1927 with an excess of liquid to guarantee a proper wetting of the glass frit 1928 that should be completely below the liquid level. The sample holder funnel 1927 is also covered with an airtight cap (not shown) to avoid evaporation and therefore a change in solution salinity. During storage stopcocks 1923 and 1924 are also accordingly closed to avoid evaporation as well as the open-ended tube 1922 airtight sealed with a cap (not shown).

*Sample preparation*

During the sample preparation, the sample is only touched with the tweezers. Discs of 6 cm diameter are cut out of the collagen material using any suitable die cutter. The samples are then stored in a closed container, e.g., a petri dish with lid, and conditioned in the controlled environment of the test room for at least 24 hours.

*Material used:*

Saline solution at a concentration of 0.9% by weight
FHFA equipment (as set out above)
Bubble level
Analytical balance with a resolution of $\pm$ 0.001 g with air draft protections.
Funnel
Tweezers
Timer

*Experiment Setup*

Before starting the experiment:

1) The caps to the open-ended tube 1922 and the sample holder funnel 1927 are removed.
2) Ensuring the stopcock 1923 is closed, the stopcock 1924 is opened to allow the air to flow out of the liquid reservoir as displaced by liquid during the refilling phase. The liquid reservoir 1921 is refilled through top end of the open-end tube 1922 with the 0.9 % Saline solution with the help of suitable means such a funnel (not shown) at the end of the filling the stopcock 1924 is closed.
If during all the experiments the liquid level would be close to the bottom 1904 of the open-ended tube 1922, before running the next sample, the liquid reservoir must be refilled repeating this step number 2.
3) The sample holder funnel 1927 is removed from the lab clamp 1914 and the excess of liquid is removed pouring it away.
4) Manually holding the sample holder funnel 1927 such that the top of the glass frit 1928 lies around 20 cm below the bottom end 1904 of the open-ended tube 1922 the stop cock 1923 is carefully open until the air liquid interface in the open-ended tube 1922 reaches the bottom end 1904 and a few bubbles of air escape from tube 1922. At this point the stop cock 1923 is closed.
5) The excess of liquid now present in the sample holder funnel 1927 is again disposed and the system is now ready to start the measurements.

For measuring the Fixed Height Frit Absorption (FHFA) at 20 cm, for each replicate:

1) The sample holder is positioned on the clamp 414 such that the top of the glass frit 1928 lies exactly 20 cm (+/- 1 mm) above the bottom end 404 of the open-ended tube 1922. To ensure a reliable measure it is checked that the glass frit 1928 is perfectly horizontal with the help of a bubble level.
2) Any remaining droplets of liquid on top of the glass frit are carefully removed by means of a filter paper of any other suitable material.
3) The sample is weighed with an analytical balance with a resolution of $\pm$ 0.001 g. The Weight is recorded as Dry Sample Weight ($W_D$) to the nearest 0.001 g when the readings on the balance become constant.
4) 4 sheets of collagen material are carefully aligned on top of each other using tweezers. This stack of 4 sheets of collagen is subsequently referred to as "sample". The sample 1930 is positioned in the center of the sample holder with the help of tweezers with particular care in not altering the orientation and relative position of each of the layers of the acquisition system.

5) The confining weight 1929 is positioned centered on the sample

6) The stopcock 1923 is opened for 30 +/- 1 seconds allowing liquid to flow in the sample and then closed again.

7) The confining weight 1929 and the sample 1930 are carefully removed from the glass frit 1928 with the help of tweezers.

8) The sample 1930 is weighed with the analytical balance with a resolution of $\pm$ 0.001 g. The Weight is recorded as 20 cm Sample Weight ($W_{20}$) to the nearest 0.001 g when the readings on the balance become constant.

The measurements of a sample are now completed, and a subsequent replicate can be measured repeating the above steps. Once terminated the series of experiment around 1 cm of liquid is added on the Sample Holder funnel 1927 to completely submerge the glass frit 1928. All the stopcocks are closed and the cap positioned according to the storage condition explained above to avoid evaporation and ensure reliability of the subsequent measurements.

*Calculations:*

The FHFA at 20 cm (FHFA $_{20}$) is defined according to the following formula:

$$FHFA_{20} = (W_{20} - W_D)/W_D \text{ and has unit of g/g.}$$

For measuring the Fixed Height Frit Absorption (FHFA) at 0 cm, for each replicate:

1) The sample holder is positioned on the clamp 1914 such that the top of the glass frit 1928 lies exactly 0 cm (+/- 1 mm) above the bottom end 404 of the open-ended tube 1922. To ensure a reliable measure it is checked that the glass frit 1928 is perfectly horizontal with the help of a bubble level.

2) Any remaining droplet of liquid on top of the glass frit are carefully removed by means of a filter paper of any other suitable material.

3) The sample is weighed with an analytical balance with a resolution of $\pm$ 0.001 g. The Weight is recorded as Dry Sample Weight ($W_D$) to the nearest 0.001 g when the readings on the balance become constant.

4) 4 sheets of collagen material are carefully aligned on top of each other using tweezers. This stack of 4 sheets of collagen is subsequently referred to as "sample". The sample 1930 is positioned in the center of the sample holder with the help of tweezers with particular care in not altering the orientation and relative position of each of the layers of the acquisition system. It is important that the topsheet facing side of each layer is facing now downwards during the experiment in the direction of the glass frit 1928, reproducing the liquid flow entrance direction correctly.

5) The confining weight 1929 is positioned centered on the sample

6) The stopcock 1923 is opened for 30 +/- 1 seconds allowing liquid to flow in the sample and then closed again.

7) The confining weight 1929 and the sample 1930 are carefully removed from the glass frit 1928 with the help of tweezers.

8) The sample 1930 is weighed with the analytical balance with a resolution of $\pm$ 0.001 g. The Weight is recorded as 0 cm Sample Weight ($W_0$) to the nearest 0.001 g when the readings on the balance become constant.

The measurements of a sample are now completed, and a subsequent replicate can be measured repeating the above steps. Once terminated the series of experiment around 1 cm of liquid is added on the Sample Holder funnel 1927 to completely submerge the glass frit 1928. All the stopcocks are closed, and the cap positioned according to the storage condition explained above to avoid evaporation and ensure reliability of the subsequent measurements.

*Calculations:*

The FHFA at 0 cm (FHFA$_0$) is defined according to the following formula:

$$FHFA_0 = (W_0 - W_D)/W_D \text{ and has unit of g/g.}$$

**IPRP Test Method**

Permeability generally refers to the quality of a porous material that causes it to allow liquids or gases to pass through it and, as such, is generally determined from the mass flow rate of a given fluid through it. The permeability of an absorbent structure is related to the material's ability to quickly acquire and transport a liquid within the structure, both of which are key features of an absorbent article. Accordingly, measuring permeability is one metric by which a material's suitability for use in absorbent articles may be assessed.

The following test is suitable for measurement of the In-Plane Radial Permeability (IPRP) of a porous material. The quantity of a saline solution (0.9% NaCl) flowing radially through an annular sample of the material under constant pressure is measured as a function of time.

Testing is performed at 23°C $\pm$ 2C° and a relative humidity 50% $\pm$ 5%. All samples are conditioned in this environment for twenty-four (24) hours before testing.

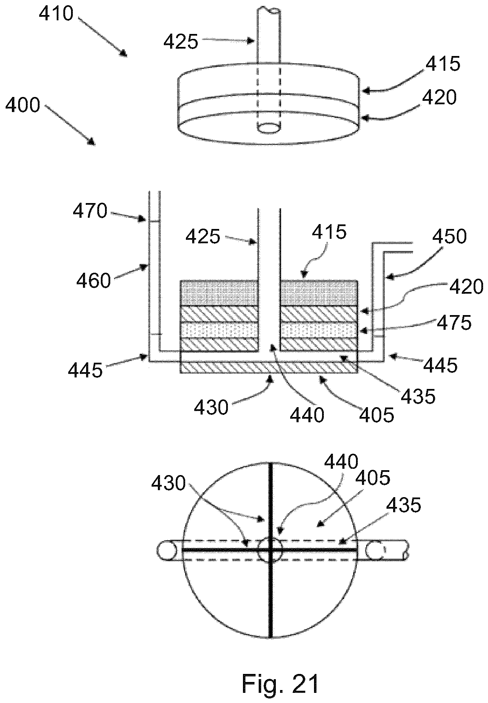

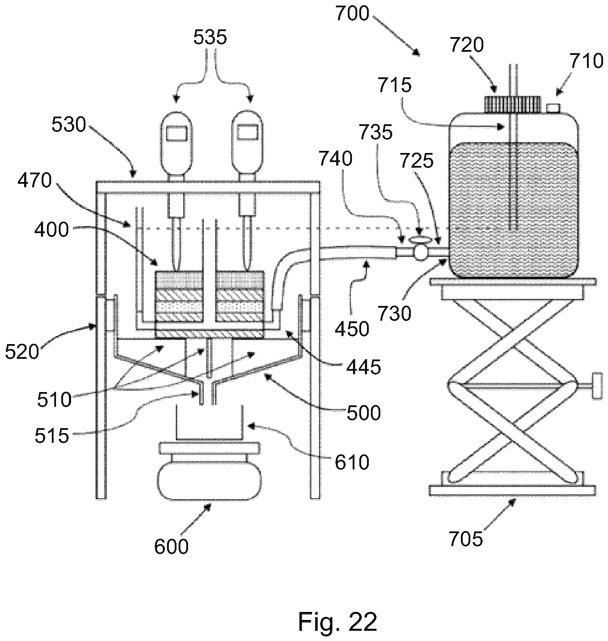

The apparatus -or parts thereof- as described below are shown in Figures 21 and 22.

The IPRP sample holder 400 is shown in FIG. 21 and comprises a cylindrical bottom plate 405, top plate 410, and cylindrical stainless-steel weight 415.

Top plate 410 comprises an annular base plate 420, which is 9 mm thick with an outer diameter of 70 mm and a tube 425 of 150 mm length fixed at the center thereof. The tube 425 has an outer diameter of 15.8 mm and an inner diameter of 12 mm. The tube is adhesively fixed into a circular 16 mm hole in the center of the base plate 420 such that the lower edge of the tube is flush with the lower surface of the base plate, as depicted in FIG. 21. The bottom plate 405 and top plate 410 are fabricated from Lexan® or equivalent. The stainless-steel weight 415 has an outer diameter of 70 mm and an inner diameter of 15.9 mm so that the weight is a close sliding fit on tube 425. The thickness of the stainless-steel weight 415 is approximately 22 mm and is adjusted so that the total weight of the top plate 410 and the stainless steel weight 415 is 687g $\pm$ 1g to provide 2.0 kPa of confining pressure during the measurement.

Bottom plate 405 is approximately 25 mm thick and has two registration grooves 430 cut into the lower surface of the plate such that each groove spans the diameter of the bottom plate and the grooves are perpendicular to each other. Each groove is 1.5 mm wide and 2 mm deep. Bottom plate 405 has a horizontal hole 435 which spans the diameter of the plate. The horizontal hole 435 has a diameter of 8 mm and its central axis is 15 mm below the upper surface of bottom plate 405. Bottom plate 405 also has a central vertical hole 440 which has a diameter of 8 mm and is 10 mm deep. The central hole 440 connects to the horizontal hole 435 to form a T-shaped cavity in the bottom plate 405. The outer portions of the horizontal hole 435 are threaded to accommodate pipe elbows 445 which are attached to the bottom plate 405 in a watertight fashion. One elbow is connected to a vertical transparent tube 460 with a total height of 175 mm measured from the bottom of bottom plate 405 (including elbow 445) and an internal diameter of 6 mm. The tube 460 is scribed with a suitable mark 470 at a height of 100 mm above the upper surface of the bottom plate 420. This is the reference for the fluid level to be maintained during the measurement. The other elbow 445 is connected to the fluid delivery reservoir 700 (described below) via a flexible tube.

A suitable fluid delivery reservoir 700 is shown in FIG. 22. Reservoir 700 is situated on a suitable laboratory jack 705 and has an air-tight stoppered opening 710 to facilitate filling of the reservoir with fluid. An open-ended glass tube 715 having an inner diameter of 10 mm extends through a port 720 in the top of the reservoir such that there is an airtight seal between the outside of the tube and the reservoir. Reservoir 700 is provided with an L-shaped delivery tube 725 having an inlet 730 that is below the surface of the fluid in the reservoir, a stopcock 735, and an outlet 740. The outlet 740 is connected to elbow 445 via flexible plastic tubing 450 (e.g., Tygon®). The internal diameter of the delivery tube 725, stopcock 735, and flexible plastic tubing 450 enable fluid delivery to the IPRP sample holder 400 at a high enough flow rate to maintain the level of fluid in tube 460 at the scribed mark 470 at all times during the measurement. The reservoir 700 has a capacity of approximately 6 liters, although larger reservoirs may be required depending on the sample thickness and permeability. Other fluid delivery systems may be employed provided that they are able to deliver the fluid to the sample holder 400 and maintain the level of fluid in tube 460 at the scribed mark 470 for the duration of the measurement.

The IPRP catchment funnel 500 is shown in FIG. 22 and comprises an outer housing 505 with an internal diameter at the upper edge of the funnel of approximately 125 mm. Funnel 500 is constructed such that liquid falling into the funnel drains rapidly and freely from spout 515. A stand with horizontal flange 520 around the funnel 500 facilitates mounting the funnel in a horizontal position. Two integral vertical internal ribs 510 span the internal diameter of the funnel and are perpendicular to each other. Each rib 510 is 1.5 mm wide and the top surfaces of the ribs lie in a horizontal plane. The funnel housing 500 and ribs 510 are fabricated from a suitably rigid material such as Lexan® or equivalent in order to support sample holder 400. To facilitate loading of the sample it is advantageous for the height of the ribs to be sufficient to allow the upper surface of the bottom plate 405 to lie above the funnel flange 520 when the bottom plate 405 is located on ribs 510. A bridge 530 is attached to flange 520 in order to mount two digital calipers 535 to measure the relative height of the stainless-steel weight 415. The digital calipers 535 have a resolution of $\pm$ 0.01 mm over a range of 25 mm. A suitable digital caliper is a Mitutoyo model 543-492B or equivalent. Each caliper is interfaced with a computer to allow height readings to be recorded periodically and stored electronically on the computer. Bridge 530 has a circular hole 22 mm in diameter to accommodate tube 425 without the tube touching the bridge.

Funnel 500 is mounted over an electronic balance 600, as shown in FIG. 22. The balance has a resolution of $\pm$ 0.01 g and a capacity of at least 1000 g. The balance 600 is also interfaced with a computer to allow the balance reading to be recorded periodically and stored electronically on the computer. A suitable balance is Mettler-Toledo model MS6002S or equivalent. A collection container 610 is situated on the balance pan so that liquid draining from the funnel spout 515 falls directly into the container 610.

The funnel 500 is mounted so that the upper surfaces of ribs 510 lie in a horizontal plane. Balance 600 and container 610 are positioned under the funnel 500 so that liquid draining from the funnel 500 falls directly into the container 610. The IPRP sample holder 400 is situated centrally in the funnel 500 with the ribs 510 located in grooves 430. The upper surface of the bottom plate 405 must be perfectly flat and level. The top plate 410 is aligned with and rests on the bottom plate 405. The stainless-steel weight 415 surrounds the tube 425 and rests on the top plate 410. Tube 425 extends vertically through the central hole in the bridge 530. Both calipers 535 are mounted firmly to the bridge 530 with the foot resting on a point on the upper surface of the stainless-steel weight 415. The calipers are set to zero in this state. The reservoir 700 is filled with 0.9% saline solution and re-sealed. The outlet 740 is connected to elbow 445 via flexible plastic tubing 450.

*Sample Preparation*

Remove the topsheet and any layer of the optional ADL from the complete absorbent article.

Retain the complete absorbent core. The backsheet can be retained to reduce the likelihood for disintegration of the absorbent core and ease handling of the sample (may not be required e.g., if the absorbent core is wrapped by a nonwoven web). However, the backsheet needs to be removed if the absorbent article has further components between the backsheet and the absorbent core. In such cases, all components need to be removed such as to isolate the absorbent core. Use die cutter to cut the sample at each location from the absorbent core in a donut shape having 70 mm - R0 exterior diameter and 12 mm - Ri interior diameter. Cut out samples from the crotch region and the back region of the absorbent core.

The annular sample 475 of material to be tested has an outer diameter of 70 mm and an inner hole diameter of 12 mm. One suitable means of cutting the sample is to use a die cutter with sharp concentric blades.

The top plate 410 is lifted enough to insert the sample 475 between the top plate and the bottom plate 405 with the sample centered on the bottom plate and the plates aligned. The stopcock 735 is opened and the level of fluid in tube 460 is set to the scribed mark 470 by adjusting the height of the reservoir 700 using the jack 705 and by adjusting the position of the tube 715 in the reservoir.

Let the saline solution flow through the sample for pre-swelling for 30 minutes, making sure that the liquid column stays at the scribed mark 470.

After the 30 min pre-swelling and when the fluid level in the tube 460 is stable at the scribed mark 470 initiate recording data from the balance and calipers by the computer. Balance readings and time elapsed are recorded every 10 seconds for five minutes. The average sample thickness B is calculated from all caliper reading between 60 seconds and 300 seconds and expressed in cm. The flow rate in grams per second is the slope calculated by linear least squares regression fit of the balance reading (dependent variable) at different times (independent variable) considering only the readings between 60 seconds and 300 seconds.

Permeability k is then calculated by the following equation:

$$k = \frac{(Q/\rho_i) \cdot \mu \cdot \ln(R_0/R_i)}{2\pi \cdot B \cdot \Delta p}$$

(1)

Where:

k is the permeability ($cm^2$);
Q is the flow rate (g/s);
$\rho_1$ is the liquid density at 20°C ($g/cm^3$);
$\mu$ is the liquid viscosity at 20 °C (Pa·s);
$R_0$ is the outer sample radius (cm);
$R_i$ is the inner sample radius (cm);
B is the average sample thickness (cm); and
$\Delta p$ is the pressure drop (Pa) calculated according to the following equation:

$$\Delta p = \left(\Delta h - \frac{B}{2}\right) \cdot g \cdot \rho_l$$

(2)

Where:

Δh is the height of the fluid level in the tube 460 from the top side of the bottom plate 405 to the mark 470; Δh is 10cm +-0.1cm- (cm);

g is the acceleration constant (m/sec$^2$); and

$\rho_1$ is the liquid density (g/cm$^3$).

**Opacity Test method**

Opacity by contrast ratio measurements are made using a 0°/45° spectrophotometer suitable for making standard CIE L*a*b* color measurements such as the Hunter ColorFlex EZ Spectrophotometer (Hunter Associates Laboratory Inc., Reston, Virginia, USA) or equivalent. The diameter of the instrument's measurement port is 30mm. Analyses are performed in a room controlled at about 23°C $\pm$ 2°C and 50%$\pm$2% relative humidity.

The instrument is calibrated per the vender instructions using standard black and white tiles provided by the instrument vendor. After calibration, the Y value of a standard white tile is measured and compared to its true value. The specified true Y value is of the standard white tile is typically in the range of 83 to 85, and the difference from true value should be 0.5 or less. The spectrophotometer is set to use the CIE XYZ color space, with a D65 standard illumination and 10° observer.

If possible, measurements are made on the lower ADS before it is integrated in an absorbent article. If this is not possible, care should be exerted when excising the lower ADS from the product not to impart any contamination or distortion to the test sample layer during the removal of the material from other layers (using cryogenic spray, such as Cyto-Freeze, Control Company, Houston, Texas, if needed). Five rectangular specimens of the lower ADS are taken such that each specimen center corresponds to the position of the loading point on the lower ADS and such that the length and width of each specimen are greater than the smallest dimension of the head.

The lower ADS specimen is positioned flat against the instrument with the outward facing surface toward the spectrophotometer's measurement port and the center of the lower ADS specimen (which corresponds to the loading point of the lower ADS) is matching with the center of the port. The specimen is further positioned such that no tears, holes or apertures are within the measurement port. The white standard tile is placed onto the opposing surface of the specimen such that it completely covers the portion of the specimen over the measurement port. A reading of XYZ values is taken, and each is recorded to the nearest 0.01. Without moving the specimen, the white plate is removed and replaced with the black standard plate. A second reading of XYZ values is taken and each is recorded to the nearest 0.01.

Opacity is calculated by dividing the Y value measured using the black tile as backing, divided by the Y value measured using the white tile as backing, then multiplying the ratio by 100.

$$Opacity \; [\%] = \frac{Y \; reading \; over \; black \; tile}{Y \; reading \; over \; white \; tile} \times 100\%$$

The five lower ADS specimens are analyzed in this way, and the Opacity of each is recorded. The arithmetic mean of the individual specimen results is calculated and reported as the Opacity in percentage to the nearest 1 %.

**Examples**

The following absorbent articles were subjected to the Urease Activity Test Method set out above:

Note: the absorbent articles (diapers) used in Examples 1-3 were handmade articles based on commercially available diapers. As the commercially available absorbent articles did not comprise any urease inhibitors, the urease inhibitor was incorporated in the samples by partially disassembling the diapers and subsequently assembling them again as described below. If the test method is applied for absorbent articles which already comprise the urease inhibitor, the subsequent incorporation of the urease inhibitor in a pre-manufactured absorbent article as described below, will not be necessary. Rather, for such absorbent articles the urease inhibitor will already have been added during manufacture of the article.

The topsheet of Pampers Baby Dry absorbent article comprised lotion applied on its body-facing surface. To avoid any possible impact of the lotion on the obtained results, the topsheet was replaced with another topsheet (as described below) in Comparative Example 1 and in Examples 1-3 below.

Comparative Example 1: Pampers Baby Dry diapers, Size 4, as commercially available in Germany in April 2021 and modified as follows:

The commercial product had a topsheet, a backsheet, an absorbent core between the topsheet and the backsheet, and an ADL with two layers between the topsheet and the absorbent core. The first layer of the ADL was provided between the topsheet and the second layer of the ADL. The first layer of the ADL is a carded nonwoven web made of PET fibers and a liquid latex binder which had been cured after application onto the fibers to solidify. The second layer of the ADL was provided between the first layer of the ADL and the absorbent core. The second layer was a layer of intra-fiber cross-linked cellulose fibers.

After the sample had been cut out from the absorbent article as is described in the test method above, the topsheet material was carefully removed (though it was not needed here, it is generally possible to use ice spray to separate the layers to ease the separation). A 12 $g/m^2$ spunbond nonwoven web made of polypropylene fibers was provided on the body-facing surface of the first layer of the ADL to replace the previous topsheet.

The samples were treated with test solution as described in the Urease Activity test method above.

Comparative Example 2

Huggies Diamond Diapers size L, available in Hongkong in April 2021. The product is said to have a "skin friendly gentle pH liner' and 'pH balance". The sample had been cut out from the absorbent article as is described in the Urease Activity test method above.

Example 1

Same as Comparative Example 1, but with Grapeseed extract (Cas no. 84929-27, Purity >95%, Batch-No 191210, supplier Sagechem) added to the absorbent article. Three different amounts of the grapeseed extract were tested, as specified in the tables below.

After the sample had been cut out from the absorbent article as is described in the Urease activity test method above, the topsheet material and the first and second layer of the ADL were carefully removed. First, the topsheet and the first and second layer of the ADL were carefully separated from the absorbent core, using a metallic ruler (topsheet and underlying first layer and second layer of the ADL remained attached to each other) removed. The absorbent core (with backsheet attached to it on the garment-facing surface) was carefully put aside.

The respective amount of grapeseed extract was added on the garment-facing surface of the second layer of the ADL. The urease inhibitor (in form of powder) was added homogeneously by sprinkling it with a metallic spatula.

Then the topsheet and first and second layer of the ADL are put back on the absorbent core by placing the second layer of the ADL with its garment-facing surface (and urease inhibitor on it) onto the body-facing surface of the absorbent core. No adhesive was applied between second layer of ADL and absorbent core.

Finally, the topsheet was carefully removed from the first layer of the ADL and replaced with the same nonwoven web that was used in Comparative Example 1.

The sample is placed in the test container and treated with test solution as described in the Urease Activity test method above.

Example 2

Same as Example 1, but instead of using grapeseed extract, Green Tea (PZN No./catalog No. 05134633, Pure Encapsulations Grüner Tee Extrakt, comprising 90 % Tee-Catechines and 70 % Epigallocatechingallate (EGCG)) was used in the same manner as the grapeseed extract used in Example 1. Three different amounts of green tea were tested, as specified in the tables below.

Example 3

Same as Example 1, but instead of using grapeseed extract, Epigallocatechin gallate (Cas. No. 989-515, 92.4% Purity, Product No. PHR1333-1G, Sigma) was used in the same manner as the grapeseed extract used in Example 1. Three different amounts of Epigallocatechin gallate were tested, as specified in the tables below.

As Control, samples similar to the samples of Comparative Example 1 were tested, however, no urease was added to the Test Solution, i.e., only Synthetic Urine were applied on the samples. The Control samples did not contain any urease inhibitor.

Table 1: Results for Example 1

|  | Ammonia [ppm] | Collagen pH | Topsheet pH |
|---|---|---|---|
| 76.4 $g/m^2$ | 0.2 | 4.6 | 6.1 |
| 38.2 $g/m^2$ | 0.5 | 4.8 | 6.7 |

(continued)

|  | Ammonia [ppm] | Collagen pH | Topsheet pH |
|---|---|---|---|
| 12.7 g/m² | 1.6 | 5.4 | 7.0 |
| Control | 0.0 | 4.3 | 5.5 |

Table 2: Results for Example 2

|  | Ammonia [ppm] | Collagen pH | Topsheet pH |
|---|---|---|---|
| 76.4 g/m² | 0.6 | 5.4 | 6.8 |
| 38.2 g/m² | 1.3 | 5.5 | 7.1 |
| 12.7 g/m² | 2.8 | 6.1 | 7.5 |
| Control | 0.0 | 4.2 | 5.3 |

Table 3: Results for Example 3

|  | Ammonia [ppm] | Collagen pH | Topsheet pH |
|---|---|---|---|
| 76.4 g/m² | 2.0 | 5.9 | 7.6 |
| 38.2 g/m² | 3.4 | 6.0 | 7.6 |
| 12.7 g/m² | 4.0 | 6.1 | 7.6 |
| Control | 0.0 | 4.4 | 5.6 |

Table 4: Results for Comparative Example 1

|  | Ammonia [ppm] | Collagen pH | Topsheet pH |
|---|---|---|---|
| No urease inhibitor | 11.4 | 6.6 | 8.5 |
| Control | 0.0 | 4.3 | 5.5 |

Table 5: Results for Comparative Example 2

|  | Ammonia [ppm] | Collagen pH | Topsheet pH |
|---|---|---|---|
| Huggies Diamond | >100.0 | 7.4 | 9.7 |
| Control | 0.0 | 4.3 | 5.5 |

The data show that for the three different urease inhibitors tested in Examples 1-3, urease inhibition can be determined in dose dependent manner (i.e. the higher amounts tested gave lower pH values for the Collagen vs. lower amounts tested). The measured levels of ammonia also reflect the effect of the different urease inhibitors. All three urease inhibitors show improved (i.e., lower) pH in collagen and in topsheet and lower levels of ammonia vs. Comparative Example 1. Comparative Example 2 shows higher ammonia level and relatively high Collagen pH and topsheet pH.

**Claims**

1. Use of an effective amount of urease inhibitor in an absorbent article to maintain the pH of the wearer's skin in a range of 4.0 to 6.0 according to the Urease Activity test method set out herein, wherein the absorbent article comprises a liquid permeable topsheet, a liquid impermeable backsheet and an absorbent core provided between the topsheet and the backsheet, wherein the urease inhibitor is provided between the topsheet and the absorbent core and/or is provided in the absorbent core;

   wherein the absorbent article has a longitudinal centerline and longitudinal dimension extending along the longitudinal centerline, and a transverse centerline perpendicular to the longitudinal centerline,
   the absorbent article comprising a front waist region with a front waist edge, a back waist region with a back waist edge, and a crotch region longitudinally extending between the front and back waist region, the absorbent article having a longitudinal dimension extending along the longitudinal centerline from the front waist edge to the rear waist edge, each region forming one third of the longitudinal dimension, and
wherein the amount of the urease inhibitor is higher in the crotch region than in the back waist region.

2.  The use of claim 1, wherein the urease inhibitor is a natural urease inhibitor.

3.  The use of claim 2, wherein the natural urease inhibitor is selected from the group consisting of green tea, extracts from green tea, grape seeds, extracts from grape seeds, and combinations thereof.

4.  The use of any of claim 1 or 2, wherein the absorbent core comprises a body-facing surface, the body-facing surface having a surface area, wherein the urease inhibitor is applied in an amount of from 3 g/m$^2$ to 200 g/m$^2$, or from 5 g/m$^2$ to 100 g/m$^2$, based on the total body-facing surface area of the absorbent core.

5.  The use of any of the preceding claims, wherein the amount of the urease inhibitor is higher in the front waist region than in the back waist region.

6.  The use of any of the preceding claims, wherein the amount of urease inhibitor is higher in the crotch region than in the front waist region.

7.  The use of any of the preceding claims, wherein the absorbent core has elongated regions with reduced caliper.

8.  The use of any of the preceding claims, wherein the urease inhibitor is further used to maintain the pH of the topsheet in a range of 5.0 to 7.5 according to the Urease Activity test method set out herein.

9.  The use of any of the preceding claims, wherein the urease inhibitor is further used to maintain the amount of ammonia below 10 ppm according to the Urease Activity test method set out herein.

10. The use of any of the preceding claims, wherein the absorbent article further comprises at least one acquisition and distribution layer (ADL) which is provided between the topsheet and the absorbent core, and wherein the urease inhibitor is applied on and/or in the ADL.

11. Absorbent article comprising a liquid permeable topsheet, a liquid impermeable backsheet and an absorbent core between the topsheet and the backsheet, wherein a urease inhibitor is/are provided between the topsheet and the absorbent core and/or is provided in the absorbent core, wherein the urease inhibitor is provided in an effective amount required in order to maintain the pH of the wearer's skin in a range of 4.0 to 6, as is measured by the test method set out herein;

wherein the absorbent article has a longitudinal centerline and longitudinal dimension extending along the longitudinal centerline, and a transverse centerline perpendicular to the longitudinal centerline,
the absorbent article comprising a front waist region with a front waist edge, a back waist region with a back waist edge, and a crotch region longitudinally extending between the front and back waist region, the absorbent article having a longitudinal dimension extending along the longitudinal centerline from the front waist edge to the rear waist edge, each region forming one third of the longitudinal dimension, and
wherein the amount of the urease inhibitor is higher in the crotch region than in the back waist region.

12. The absorbent article of claim 11, wherein the urease inhibitor is a natural urease inhibitor.

13. The absorbent article of claim 12, wherein the natural urease inhibitor is selected from the group consisting of green tea, extracts from green tea, grape seeds, extracts from grape seeds, and combinations thereof.

14. The absorbent article of any of claims 11 to 13, wherein the absorbent article optionally comprises at least one acquisition and distribution layer (ADL) which is provided in between the topsheet and the absorbent core, and wherein at least a portion of the urease inhibitor is applied a) between the topsheet and the optional ADL, b) in the optional ADL, c) between the optional ADL and the absorbent core, d) in the absorbent core; or e) any combination of a) to d).

15. The absorbent article of any of claims 11 to 14, wherein the urease inhibitor is applied in an amount of from 3 g/m$^2$ to

200 g/m$^2$, or from 5 g/m$^2$ to 100 g/m$^2$, based on the total body-facing surface area of the absorbent core.

16. The absorbent article of any of claims 11 to 15, wherein the amount of the urease inhibitor is higher in the front waist region than in the back waist region.

17. The absorbent article of any of claims 11 to 16, wherein the amount of urease inhibitor is higher in the crotch region than in the front waist region.

18. The absorbent article of any of claims 11 to 17, wherein the absorbent core has elongated regions with reduced caliper.

**Patentansprüche**

1. Verwendung einer wirksamen Menge eines Ureaseinhibitors in einem Absorptionsartikel, um den pH-Wert der Haut des Trägers in einem Bereich von 4,0 bis 6,0 gemäß dem hierin beschriebenen Prüfverfahren für Ureaseaktivität zu halten, wobei der Absorptionsartikel eine flüssigkeitsdurchlässige Oberschicht, eine flüssigkeitsundurchlässige Unterschicht und einen Absorptionskern, der zwischen der Oberschicht und der Unterschicht bereitgestellt ist, umfasst, wobei der Ureaseinhibitor zwischen der Oberschicht und dem Absorptionskern bereitgestellt ist und/oder in dem Absorptionskern bereitgestellt ist,

   wobei der Absorptionsartikel eine Mittellinie in Längsrichtung und eine Längsabmessung aufweist, die sich entlang der Mittellinie in Längsrichtung erstreckt, und eine Quermittellinie, die lotrecht zu der Mittellinie in Längsrichtung verläuft,
   der Absorptionsartikel umfassend einen vorderseitigen Taillenbereich mit einem vorderseitigen Taillenrand, einen rückseitigen Taillenbereich mit einem rückseitigen Taillenrand und einen Schrittbereich, der sich in Längsrichtung zwischen der vorderseitigen und der rückseitigen Taillenbereich erstreckt, wobei der Absorptionsartikel eine Längsabmessung aufweist, die sich entlang der Mittellinie in Längsrichtung von dem vorderseitigen Taillenrand zu dem rückseitigen Taillenrand erstreckt, wobei jeder Bereich ein Drittel der Längsabmessung ausbildet, und wobei die Menge des Ureaseinhibitors in dem Schrittbereich höher als in dem hinteren Taillenbereich ist.

2. Verwendung nach Anspruch 1, wobei der Ureaseinhibitor ein natürlicher Ureaseinhibitor ist.

3. Verwendung nach Anspruch 2, wobei der natürliche Ureaseinhibitor aus der Gruppe ausgewählt ist, bestehend aus grünem Tee, Extrakten aus grünem Tee, Traubenkernen, Extrakten aus Traubenkernen und Kombinationen davon.

4. Verwendung nach Anspruch 1 oder 2, wobei der Absorptionskern eine körperseitige Oberfläche umfasst, wobei die körperseitige Oberfläche einen Oberflächenbereich aufweist, wobei der Ureaseinhibitor in einer Menge von 3 g/m$^2$ bis 200 g/m$^2$ oder von 5 g/m$^2$ bis 100 g/m$^2$, basierend auf der gesamten körperseitigen Oberfläche des Absorptionskerns aufgebracht ist.

5. Verwendung nach einem der vorstehenden Ansprüche, wobei die Menge des Ureaseinhibitors in dem vorderseitigen Taillenbereich höher als in dem rückseitigen Taillenbereich ist.

6. Verwendung nach einem der vorstehenden Ansprüche, wobei die Menge des Ureaseinhibitors in dem Schrittbereich höher als in dem vorderseitigen Taillenbereich ist.

7. Verwendung nach einem der vorstehenden Ansprüche, wobei der Absorptionskern langgestreckte Bereiche mit reduzierter Dicke aufweist.

8. Verwendung nach einem der vorstehenden Ansprüche, wobei der Ureaseinhibitor ferner verwendet wird, um den pH-Wert der Oberschicht in einem Bereich von 5,0 bis 7,5 gemäß dem hierin beschriebenen Prüfverfahren für Ureaseaktivität zu halten.

9. Verwendung nach einem der vorstehenden Ansprüche, wobei der Ureaseinhibitor ferner verwendet wird, um die Ammoniakmenge unter 10 ppm gemäß dem hierin beschriebenen Prüfverfahren für Ureaseaktivität zu halten.

10. Verwendung nach einem der vorstehenden Ansprüche, wobei der Absorptionsartikel ferner wenigstens eine Aufnahme- und Verteilungsschicht (ADL) umfasst, die zwischen der Oberschicht und dem Absorptionskern bereitgestellt ist, und wobei der Ureaseinhibitor auf und/oder in der ADL aufgebracht ist.

11. Absorptionsartikel, umfassend eine flüssigkeitsdurchlässige Oberschicht, eine flüssigkeitsundurchlässige Unterschicht und einen Absorptionskern zwischen der Oberschicht und der Unterschicht, wobei ein Ureaseinhibitor zwischen der Oberschicht und dem Absorptionskern bereitgestellt ist/sind und/oder in dem Absorptionskern bereitgestellt ist/sind, wobei der Ureaseinhibitor in einer wirksamen Menge bereitgestellt ist, die erforderlich ist, um den pH-Wert der Haut des Trägers in einem Bereich von 4,0 bis 6 zu halten, wie gemessen mit dem hierin beschriebenen Prüfverfahren;

wobei der Absorptionsartikel eine Mittellinie in Längsrichtung und eine Längsabmessung aufweist, die sich entlang der Mittellinie in Längsrichtung erstreckt, und eine Quermittellinie, die lotrecht zu der Mittellinie in Längsrichtung verläuft,
der Absorptionsartikel umfassend einen vorderseitigen Taillenbereich mit einem vorderseitigen Taillenrand, einen rückseitigen Taillenbereich mit einem rückseitigen Taillenrand und einen Schrittbereich, der sich in Längsrichtung zwischen der vorderseitigen und der rückseitigen Taillenbereich erstreckt, wobei der Absorptionsartikel eine Längsabmessung aufweist, die sich entlang der Mittellinie in Längsrichtung von dem vorderseitigen Taillenrand zu dem rückseitigen Taillenrand erstreckt, wobei jeder Bereich ein Drittel der Längsabmessung ausbildet, und wobei die Menge des Ureaseinhibitors in dem Schrittbereich höher als in dem hinteren Taillenbereich ist.

12. Absorptionsartikel nach Anspruch 11, wobei der Ureaseinhibitor ein natürlicher Ureaseinhibitor ist.

13. Absorptionsartikel nach Anspruch 12, wobei der natürliche Ureaseinhibitor aus der Gruppe ausgewählt ist, bestehend aus grünem Tee, Extrakten aus grünem Tee, Traubenkernen, Extrakten aus Traubenkernen und Kombinationen davon.

14. Absorptionsartikel nach einem der Ansprüche 11 bis 13, wobei der Absorptionsartikel optional wenigstens eine Aufnahme- und Verteilungsschicht (ADL) umfasst, die zwischen der Oberschicht und dem Absorptionskern bereitgestellt ist, und wobei wenigstens ein Teil des Ureaseinhibitors aufgebracht ist: a) zwischen der Oberschicht und der optionalen ADL, b) in der optionalen ADL, c) zwischen der optionalen ADL und dem Absorptionskern, d) in dem Absorptionskern; oder e) in einer beliebigen Kombination von a) bis d).

15. Absorptionsartikel nach einem der Ansprüche 11 bis 14, wobei der Ureaseinhibitor in einer Menge von 3 g/m$^2$ bis 200 g/m$^2$ oder von 5 g/m$^2$ bis 100 g/m$^2$, basierend auf der gesamten körperseitigen Oberfläche des Absorptionskerns aufgebracht ist.

16. Absorptionsartikel nach einem der Ansprüche 11 bis 15, wobei die Menge des Ureaseinhibitors in dem vorderseitigen Taillenbereich höher als in dem rückseitigen Taillenbereich ist.

17. Absorptionsartikel nach einem der Ansprüche 11 bis 16, wobei die Menge des Ureaseinhibitors in dem Schrittbereich höher als in dem vorderseitigen Taillenbereich ist.

18. Absorptionsartikel nach einem der Ansprüche 11 bis 17, wobei der Absorptionskern langgestreckte Bereich mit reduzierter Dicke aufweist.


**Revendications**

1. Utilisation d'une quantité efficace d'inhibiteur d'uréase dans un article absorbant pour maintenir le pH de la peau du porteur dans une plage de 4,0 à 6,0 selon la méthode d'essai de l'activité uréase décrite ici, dans laquelle l'article absorbant comprend une feuille de dessus perméable aux liquides, une feuille de fond imperméable aux liquides et une âme absorbante fournie entre la feuille de dessus et la feuille de fond, dans laquelle l'inhibiteur d'uréase est fourni entre la feuille de dessus et l'âme absorbante et/ou est fourni dans l'âme absorbante ;

dans laquelle l'article absorbant a une ligne centrale longitudinale et une dimension longitudinale s'étendant le long de la ligne centrale longitudinale, et une ligne centrale transversale perpendiculaire à la ligne centrale longitudinale,

l'article absorbant comprenant une région de ceinture avant avec un bord de ceinture avant, une région de ceinture arrière avec un bord de ceinture arrière, et une région d'entrejambe s'étendant longitudinalement entre la région de ceinture avant et la région de ceinture arrière, l'article absorbant ayant une dimension longitudinale s'étendant le long de la ligne centrale longitudinale du bord de ceinture avant au bord de ceinture arrière, chaque région formant un tiers de la dimension longitudinale, et dans laquelle la quantité de l'inhibiteur d'uréase est plus élevée dans la région d'entrejambe que dans la région de ceinture arrière.

2. Utilisation selon la revendication 1, dans laquelle l'inhibiteur d'uréase est un inhibiteur naturel d'uréase.

3. Utilisation selon la revendication 2, dans laquelle l'inhibiteur naturel d'uréase est choisi dans le groupe constitué de thé vert, extraits de thé vert, pépins de raisin, extraits de pépins de raisin, et combinaisons de ceux-ci.

4. Utilisation selon l'une quelconque de la revendication 1 ou 2, dans laquelle l'âme absorbante comprend une surface faisant face au corps, la surface faisant face au corps ayant une surface, dans laquelle l'inhibiteur d'uréase est appliqué en une quantité allant de 3 g/m$^2$ à 200 g/m$^2$, ou de 5 g/m$^2$ à 100 g/m$^2$, sur la base de la surface totale faisant face au corps de l'âme absorbante.

5. Utilisation selon l'une quelconque des revendications précédentes, dans laquelle la quantité de l'inhibiteur d'uréase est plus élevée dans la région de ceinture avant que dans la région de ceinture arrière.

6. Utilisation selon l'une quelconque des revendications précédentes, dans laquelle la quantité d'inhibiteur d'uréase est plus élevée dans la région d'entrejambe que dans la région de ceinture avant.

7. Utilisation selon l'une quelconque des revendications précédentes, dans laquelle l'âme absorbante a des régions allongées à épaisseur réduite.

8. Utilisation selon l'une quelconque des revendications précédentes, dans laquelle l'inhibiteur d'uréase est en outre utilisé pour maintenir le pH de la feuille de dessus dans une plage de 5,0 à 7,5 selon la méthode d'essai de l'activité uréase décrite ici.

9. Utilisation selon l'une quelconque des revendications précédentes, dans laquelle l'inhibiteur d'uréase est en outre utilisé pour maintenir la quantité d'ammoniac en dessous de 10 ppm selon la méthode d'essai de l'activité uréase décrite ici.

10. Utilisation selon l'une quelconque des revendications précédentes, dans laquelle l'article absorbant comprend en outre au moins une couche d'acquisition et de distribution (ADL) qui est fournie entre la feuille de dessus et l'âme absorbante, et dans laquelle l'inhibiteur d'uréase est appliqué sur et/ou dans l'ADL.

11. Article absorbant comprenant une feuille de dessus perméable aux liquides, une feuille de fond imperméable aux liquides et une âme absorbante entre la feuille de dessus et la feuille de fond, dans lequel un inhibiteur d'uréase est fourni entre la feuille de dessus et l'âme absorbante et/ou est fourni dans l'âme absorbante, dans lequel l'inhibiteur d'uréase est fourni en une quantité efficace nécessaire pour maintenir le pH de la peau du porteur dans une plage de 4,0 à 6, tel que mesuré par la méthode d'essai décrite ici ;

dans lequel l'article absorbant a une ligne centrale longitudinale et une dimension longitudinale s'étendant le long de la ligne centrale longitudinale, et une ligne centrale transversale perpendiculaire à la ligne centrale longitudinale,
l'article absorbant comprenant une région de ceinture avant avec un bord de ceinture avant, une région de ceinture arrière avec un bord de ceinture arrière, et une région d'entrejambe s'étendant longitudinalement entre la région de ceinture avant et la région de ceinture arrière, l'article absorbant ayant une dimension longitudinale s'étendant le long de la ligne centrale longitudinale du bord de ceinture avant au bord de ceinture arrière, chaque région formant un tiers de la dimension longitudinale, et dans lequel la quantité de l'inhibiteur d'uréase est plus élevée dans la région d'entrejambe que dans la région de ceinture arrière.

12. Article absorbant selon la revendication 11, dans lequel l'inhibiteur d'uréase est un inhibiteur d'uréase naturel.

13. Article absorbant selon la revendication 12, dans lequel l'inhibiteur naturel d'uréase est choisi dans le groupe constitué de thé vert, extraits de thé vert, pépins de raisin, extraits de pépins de raisin, et combinaisons de ceux-ci.

**14.** Article absorbant selon l'une quelconque des revendications 11 à 13, dans lequel l'article absorbant comprend facultativement au moins une couche d'acquisition et de distribution (ADL) qui est fournie entre la feuille de dessus et l'âme absorbante, et dans lequel au moins une partie de l'inhibiteur d'uréase est appliquée a) entre la feuille de dessus et l'ADL facultative, b) dans l'ADL facultative, c) entre l'ADL facultative et l'âme absorbante, d) dans l'âme absorbante ; ou e) toute combinaison de a) à d).

**15.** Article absorbant selon l'une quelconque des revendications 11 à 14, dans lequel l'inhibiteur d'uréase est appliqué en une quantité allant de 3 g/m$^2$ à 200 g/m$^2$, ou de 5 g/m$^2$ à 100 g/m$^2$, sur la base de la surface totale faisant face au corps de l'âme absorbante.

**16.** Article absorbant selon l'une quelconque des revendications 11 à 15, dans lequel la quantité de l'inhibiteur d'uréase est plus élevée dans la région de ceinture avant que dans la région de ceinture arrière.

**17.** Article absorbant selon l'une quelconque des revendications 11 à 16, dans lequel la quantité d'inhibiteur d'uréase est plus élevée dans la région d'entrejambe que dans la région de ceinture avant.

**18.** Article absorbant selon l'une quelconque des revendications 11 à 17, dans lequel la structure d'âme absorbante a des régions allongées à épaisseur réduite.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17A

Fig. 17B

Fig. 17C

Fig. 17D

Fig. 17E

Fig. 18A

Fig. 18B

EP 4 129 259 B1

Fig. 19

Fig. 20

Fig. 21

Fig. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2020060897 A
- US 2013096526 A
- CN 110946712
- US 2003082219 A
- KR 20190002144
- US 20140005020
- US 9421137 B
- US 9011402 B, Roe
- US 20130211363
- US 7901393 B
- US 9498 A
- US 389 A
- US 6645190 B
- US 8747379 B
- US 8372052 B
- US 8361048 B
- US 6761711 B
- US 6817994 B
- US 8007485 B
- US 7862550 B
- US 6969377 B
- US 7497851 B
- US 6849067 B
- US 6893426 B
- US 6953452 B
- US 6840928 B
- US 8579876 B
- US 7682349 B
- US 7156833 B
- US 7201744 B
- US 20070219521 A1
- US 20070219521
- US 8703450 B
- US 9630901 B
- US 9822197 B
- US 20110139657
- US 20110139658
- US 20110152812
- US 20160206774
- US 9169366 B
- US 20190192723 A

### Non-patent literature cited in the description

- *CHEMICAL ABSTRACTS*, 84929-27
- *CHEMICAL ABSTRACTS*, 989-515